# EUROPÄISCHE PATENTSCHRIFT

(11) **EP 1 599 435 B1**
(45) Veröffentlichungstag und Bekanntmachung des Hinweises auf die Patenterteilung: **07.03.2012**
(21) Anmeldenummer: 03756471.3
(22) Anmeldetag: 01.10.2003
(51) Int. Cl.: C07C 43/10, C07C 43/12, C07C 41/03, C11D 3/20

(54) **UMSETZUNGSPRODUKTE VON 2-PROPYLHEPTANOL MIT 1-HALOGEN-2,3-EPOXYPROPANEN UND 1-HYDROXY-2,3-EPOXYPROPAN**
REACTION PRODUCTS OF 2-PROPYLHEPTANOL WITH 1-HALOGEN-2,3-EPOXYPROPANES AND 1-HYDROXY-2,3-EPOXYPROPANE
PRODUITS ISSUS DE LA REACTION DE 2-PROPYLHEPTANOL, DE 1-HALOGENE-2,3-EPOXYPROPANES ET DE 1-HYDROXY-2,3-EPOXYPROPANE

(30) Priorität: 01.10.2002 DE 10246140
(43) Veröffentlichungstag der Anmeldung: 30.11.2005
(73) Patentinhaber: BASF SE, 67056 Ludwigshafen (DE)
(72) Erfinder: WULFF, Christian, 68163 Mannheim (DE); NÖRENBERG, Ralf, 55218 Ingelheim (DE); KLUGE, Michael, 67071 Ludwigshafen (DE); WAGNER, Norbert, 67112 Mutterstadt (DE)
(86) Internationale Anmeldenummer: PCT/EP2003/010887
(87) Internationale Veröffentlichungsnummer: WO 2004/031111

(56) Entgegenhaltungen:
- WO-A-97/22651
- DE-A- 2 535 778

## Beschreibung

Die vorliegende Erfindung bezieht sich auf Umsetzungsprodukte von 2-Propylheptanol mit 1-Halogen-2,3-epoxypropanen und 1-Hydroxy-2,3-epoxypropan (Glycidol), Verfahren zu deren Herstellung und deren Verwendung als Co-Tenside, Reinigungstenside beziehungsweise Verdickungsmittel.

Tenside sind sogenannte amphiphile Moleküle, die in ihrer Molekülstruktur einen hydrophoben und einen hydrophilen Anteil aufweisen. Durch diese Eigenschaft können Tenside Grenzflächenfilme und sogenannte Micellen ausbilden. Dabei handelt es sich um Aggregate aus Tensiden, die sich in wässrigen Lösungen bilden und unterschiedliche Gestalt (Kugeln, Stäbchen, Scheiben) annehmen können. Micellen bilden sich oberhalb einer bestimmten Konzentration, der sogenannten kritischen Micell-Bildungskonzentration (KMK). Weiterhin besitzen amphiphile Moleküle die Eigenschaft, Grenzflächenfilme zwischen hydrophoben und hydrophilen Phasen auszubilden und so beispielsweise emulgierend oder schäumend zu wirken.

Co-Tenside haben ebenfalls amphiphile Eigenschaften, die jedoch nicht ausreichen, um alleine Micellen und Grenzflächenfilme bilden zu können. Sie werden jedoch zwischen den Tensiden eingelagert und bewirken eine Steigerung der Packungsdichte der Amphiphilen (Tenside und Co-Tenside) in den von diesen geformten Gebilden wie Micellen oder Grenzflächen. Dadurch erniedrigt sich neben der kritischen Micellbildungskonzentration und der Oberflächenspannung auch die Grenzflächenspannung zwischen der wässrigen Tensidlösung und unpolaren Substanzen wie beispielsweise Ölen, so dass die Aufnahmekapazität des Tensidsystems für diese Stoffe bis hin zur Bildung von Mikroemulsionen steigt. Es resultieren ein höheres Solubilisier- und Emulgiervermögen, eine höhere Reinigungskapazität sowie eine erhöhte Stabilität der Emulsionen und Schäume. Bei Einsatz von Co-Tensiden können Micellen bereits bei deutlich niedrigerer Tensid-Konzentration ausgebildet werden.

Weitere Effekte, die durch den Einsatz der Co-Tenside und die dadurch verstärkte Aggregationstendenz der Amphiphile hervorgerufen werden, sind bekannt. Dies ist zum einen die Aggregationstransformation von sphärischen zu anisometrischen micellaren Assoziaten. Diese Strukturänderung der Micellen hat Auswirkungen auf die Rheologie der die Micellen enthaltenen Lösungen, insbesondere in verdünnten Lösungen. Gleichzeitig tritt im Phasendiagramm eine Verschiebung vorliegender flüssigkristalliner Strukturen zu niedrigeren Konzentrationen auf, wodurch eine bevorzugte Bildung von Gelphasen mit hoher Packungsdichte zu beobachten ist. Als Folge davon treten bereits bei Konzentrationen von deutlich < 10 Gew.-% lamellare Micellstrukturen auf, die sonst erst bei wesentlich höheren Konzentrationen beobachtet werden. Ein weiteres interessantes Phänomen ist das Ausbilden, neben den bekannten flüssigkristallinen Gelphasen, von neuen Überstrukturen, die interessante anwendungstechnische Eigenschaften aufweisen. Von besonderem Interesse sind hierbei vesikuläre Phasen sowie sogenannte L₃-Phasen, die schwammartig ausgebildet sind und mikroemulsionsähnliche Eigenschaften aufweisen. Sie können in verdünnten Konzentrationsbereichen zur Einstellung der Viskosität genutzt werden. Solche assoziierenden Verdicker haben die Eigenschaft, bei hoher Scherbeanspruchung dünnflüssig zu werden und in Ruhe die Viskosität erneut aufzubauen. In der Anwendung bedeutet das gutes Suspensionsvermögen bei gleichzeitiger Gießbarkeit und bei der Produktion eine größere Stabilität der Formulierung als bei den üblicheren polymeren Verdickungsmitteln.

Aus dem Stand der Technik sind eine Anzahl von Verbindungen bzw. Verbindungsklassen bekannt, die als Co-Tenside geeignet sind.

C₅-C₁₀-Alkohole zeigen vorteilhafte Eigenschaften, kommen jedoch häufig wegen ihres charakteristischen Geruchs nicht zum Einsatz.

Niedrig ethoxilierte Alkohole wie beispielsweise niedrig ethoxilierte Laurylalkoholethoxilate, Diethylenglykolmonohexylether oder Propylenglykolbutylether können in einigen Tensidsystemen zu verbesserter Emulgierleistung oder Schaumstabilität führen, weisen aber für Tensidformulierungen mit hohem Aniontensidgehalt eine zu geringe Polarität der Kopfgruppe auf.

Fettsäureethanolamine werden beispielsweise zur Einstellung der Viskosität in Shampoos eingesetzt. Sie stehen jedoch im Verdacht, Nitrosamine auszubilden.

G. J. Smith beschreibt in Seifen, Ölen, Fette, Wachse, 105 (1979, Seiten 319 ff und 345 ff) den Einsatz von Alkylaminoxiden als Co-Tensid in verschiedenen Anwendungen. Auch sie stehen im Verdacht Nitrosamine zu enthalten. Durch eine ausgefüllte, aufwendige Herstellungstechnologie kann das weitgehend vermieden werden.

Analog den Aminoxiden können auch andere zwitterinonische Tenside, wie z. B. Sulfo- oder Carboxylammonio-betaine als Co-Tensid verwendet werden. Bei diesen Produkten ist die Ausbildung von Gelphasen sehr schwach ausgeprägt. Statt dessen haben sie aber den Anwendungsvorteil, dass die Hautreizwirkung von entsprechenden Tensidmischungen abgelenkt wird.

Die WO 98/00418 offenbart Alkylencarbonate, die mit Alkylgruppen substituiert sind, und deren Einsatz als Co-Tenside. WO 97/22 651 betrifft nichtgeladene oberflächenaktive Substanzen.

WO 97/04059 betrifft Reinigungsmittelzusammensetzungen, die einen analephotropischen negativ geladenen Komplex enthalten, der aus mindestens einem anionischen Tensid und einem damit komplexierten Alkylencarbonat aufgebaut ist. Zusätzlich können die Reinigungsmittelzusammensetzungen gegebenenfalls ein Co-Tensid, einen wasserunlöslichen Kohlenwasserstoff, ein Parfum, eine Lewis-Base oder ein neutrales Polymer enthalten. Das Alkylencarbonat weist einen C₄- bis C₁₄-Alkylrest auf.

Bei den bis jetzt bekannten Anwendungen schwankt das eingesetzte Verhältnis von Co-Tensiden zu Tensiden je nach Anwendung von ca. 1 : 20 bis 1 : 2. In einigen Fällen, wie z. B. Alkylaminoxiden, kann das Co-Tensid auch höher konzentriert sein. Des weiteren sind die zur Herstellung der Co-Tenside eingesetzten Ausgangsstoffe oft teuer.

Der vorliegenden Erfindung liegt die Aufgabe zugrunde, als Co-Tenside, Reinigungstenside beziehungsweise Verdickungsmittel geeignete Verbindungen zur Verfügung zu stellen, die die genannten Nachteile nicht aufweisen, insbesondere eine sehr gute Kosteneffizienz und Effektivität zeigen sowie umweltverträglich und frei von Risiken für den Menschen sind. Insbesondere ist es die Aufgabe der vorliegenden Erfindung einen kostengünstigen Zugang zu Alkylglycidolcarbonaten zu ermöglichen, wobei diese Alkylglycidolcarbonate eine besonders gute Performance als Co-Tensid zeigen.

Diese Aufgabe wird gelöst durch Verbindungen der allgemeinen Formel I 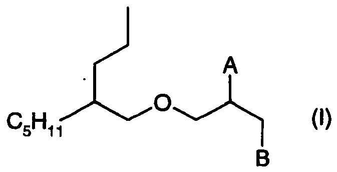 worin
- A: eine OH-Gruppe und
- B: eine OH-Gruppe (Verbindung Ia) oder ein Halogenatom, bevorzugt ein Cl-Atom, (Verbindung Ib) ist, oder
- A und B: gemeinsam einen Rest der Formel 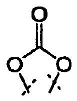 darstellen (Verbindung Ic), d.h. Teil desselben cyclischen Carbonatrings sind, oder gemeinsam einen Rest der Formel 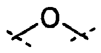 darstellen (Verbindung Id), d.h. Teil desselben Epoxidrings sind,
und
- C₅H₁₁: ein unverzeigter oder verzweigter C₅H₁₁-Alkylzest oder ein Gemisch aus unverzweigten und verzweigten C₅H₁₁-Alkylresten ist,
wobei 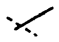 eine Bindung zu einem weiteren Kohlenstoffatom bedeutet.

Die Verbindungen der Formel I umfassen verschiedene Verbindungsklassen, die bei der Herstellung von Alkylgylcidolcarbonaten der Formel Ic, d.h. Verbindungen der Formel I, worin A und B gemeinsam einen Rest der Formel 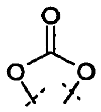 darstellen, d.h. Teil desselben cyclischen Carbonatrings sind, gebildet werden. Die Alkylglycidolcarbonate der Formel I eignen sich hervorragend für den Einsatz als Co-Tenside in den üblichen, dem Fachmann bekannten Wasch- und Reinigungsformulierungen.

Auch die weiteren erfindungsgemäßen Verbindungen weisen vorteilhafte Eigenschaften auf. So eignen sich Verbindungen der Formel Ia, worin A und B jeweils eine OH-Gruppe bedeuten, hevorragend als Reinigungstenside in üblichen, dem Fachmann bekannten Wasch- und Reinigungsformulierungen. Sie lösen z. B. Ölverschmutzungen sehr schnell und gründlich ab.

Im Folgenden sind die erfindungsgemäßen Verbindungen Ia, Ib, Ic und Id dargestellt:

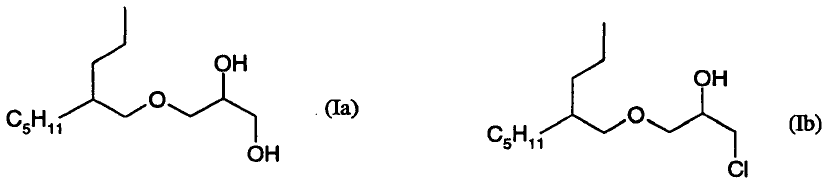

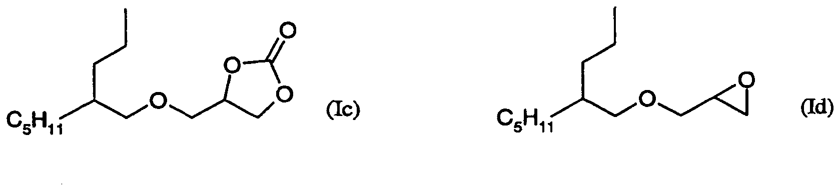

Der Rest C₅H₁₁ in den Verbindungen der Formel I kann ein unverzweigter oder verzweigter C₅H₁₁-Alkylrest sein oder ein Gemisch verschiedener unverzweigter oder verzweigter C₅H₁₁-Alkylreste. Bevorzugt ist der Rest C₅H₁₁ ein Gemisch aus unverzweigten und verzweigten C₅H₁₁-Alkylresten, das 70 bis 99 Gew.-% eines unverzweigten n-C₅H₁₁-Alkylrests und 1 bis 30 Gew.-% eines verzweigten C₅H₁₁-Alkylrests, besonders bevorzugt C₂H₅CH(CH₃)CH₂ und/oder CH₃CH(CH₃)CH₂CH₂, umfasst Besonders bevorzugt ist der Rest C₅H₁₁ ein Gemisch aus unverzweigten und verzweigten C₅H₁₁-Alkylresten, das etwa 90 Gew.-% eines unverzweigten n-C₅H₁₁-Alkylrests und etwa 10 Gew.-% C₂H₅CH(CH₃)CH₂ und/oder CH₃CH(CH₃)CH₂CH₂ umfasst.

Die erfindungsgemäßen Verbindungen können durch Umsetzung einer Hydrophobkomponente mit 1-Halogen-2,3-epoxypropan, bevorzugt Epichlorhydrin, oder 1-Hydroxy-2,3-epoxypropan - und gegebenenfalls weitere daran anschließende Umsetzungen - erhalten werden.

Die bei der Herstellung der erfindungsgemäßen Verbindungen eingesetzte Hydrophobkomponente ist 2-Propylheptanol. Dabei handelt es sich um einen besonders preisgünstigen Alkohol. Besonders bevorzugt wird ein Gemisch aus 2-Propylheptanol mit isomeren Alkoholen eingesetzt. Dieses Gemisch enthält im allgemeinen 70 bis 99 Gew.-% 2-Propylheptanol und 1 bis 30 Gew.-% weiterer verzweigter isomerer Alkohole, bevorzugt 4-Methyl-2-propyl-hexanol und/oder 5-Methyl-2-propyl-hexanol. Ganz besonders bevorzugt wird ein technisch anfallendes Gemisch eingesetzt, das etwa 90 Gew.-% 2-Propylheptanol und etwa 10 Gew.-% 4-Methyl-2-propyl-hexanol und/oder 5-Methyl-2-propyl-hexanol enthält.

Bevorzugt ist eine Verbindung der Formel Ic, worin A und B gemeinsam einen Rest der Formel 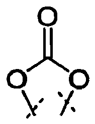 darstellen, d.h. Teil desselben cyclischen Carbonatrings sind. Bei dieser Verbindung handelt es sich um ein Alkylglycidolcarbonat, das - wie vorstehend erwähnt - hervorragend als Co-Tensid geeignet ist.

In einer weiteren bevorzugten Ausführungsform handelt es sich bei der erfindungsgemäßen Verbindung der allgemeinen Formel Ia um eine Verbindung, worin sowohl A als auch B eine OH-Gruppe sind. Bei dieser Verbindung handelt es sich um ein Diol, das - wie vorstehend erwähnt - hervorragend als Reinigungstensid geeignet ist.

Das erfindungsgemäße Diol der Formel Ia kann wiederum mit 1-Halogen-2,3-epoxypropan, bevorzugt Epichlorhydrin, oder 1-Hydroxy-2,3-epoxypropan umgesetzt werden. Besonders bevorzugt ist hier die Verwendung von 1-Hydroxy-2,3-epoxypropan. Ein weiterer Gegenstand der vorliegenden Erfindung ist daher ein Umsetzungsprodukt (IIIa), herstellbar durch Umsetzung eines Moläquivalents des erfindungsgemäßen Diols mit 0 bis 10, bevorzugt 0 bis 4, besonders bevorzugt 0 bis 1.5 Moläquivalenten 1-Halogen-2,3-epoxypropan, bevorzugt Epichlorhydrin, oder 1-Hydroxy-2,3-epoxypropan. Dieses Umsetzungsprodukt enthält dann Moleküle, die durchschnittlich insgesamt bevorzugt 1 bis 11, besonders bevorzugt 1 bis 5, ganz besonders bevorzugt 1 bis 2,5 Struktureinheiten der folgenden Formel A: 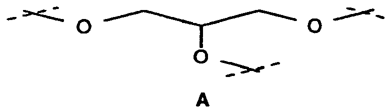 und ggf. der folgenden Formel B enthalten:

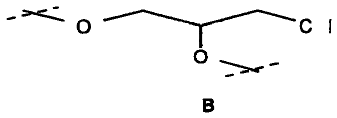

Exemplarisch dargestellt sind hier die Formeln möglicher Umsetzungsprodukte von einem Molekül des Diols mit einem Molekül Epichlorhydrin (IIa) und einem Molekül des Diols mit einem Molekül 1-Hydroxy-2,3-epoxypropan (IIb). Im letzteren Fall kann auch die sekundäre OH-Funktion des Diols (Ia) mit dem Epoxid reagieren, so dass eine andere Struktur als IIb resultiert.

In einer weiteren bevorzugten Ausführungsform handelt es sich bei der erfindungsgemäßen Verbindung der allgemeinen Formel Ib um eine Verbindung, worin A eine OH-Funktion und B ein Cl-Atom ist. Bei dieser Verbindung handelt es sich um ein Chlorhydrin, das als Edukt zur Synthese anderer erfindungsgemäßer Verbindungen eingesetzt werden kann.

Das erfindungsgemäße Chlorhydrin Ib kann wiederum mit 1-Halogen-2,3-epoxypropan, bevorzugt Epichlorhydrin, oder 1-Hydroxy-2,3-epoxypropan umgesetzt werden. Besonders bevorzugt ist hier die Verwendung von Epichlorhydrin. Ein weiterer Gegenstand der vorliegenden Erfindung ist daher ein Umsetzungsprodukt IIIb, herstellbar durch Umsetzung eines Moläquivalents des erfindungsgemäßen Chlorhydrins Ib mit 0 bis 10, bevorzugt 0 bis 4, besonders bevorzugt 0 bis 1.5 Moläquivalenten 1-Halogen-2,3-epoxypropan, bevorzugt Epichlorhydrin, oder 1-Hydroxy-2,3-epoxypropan. Dieses Umsetzungsprodukt enthält insgesamt bevorzugt 1 bis 11, besonders bevorzugt 1 bis 5, ganz besonders bevorzugt 1 bis 2,5 Struktureinheiten der folgenden Formel B: 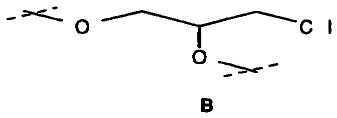 und ggf. der folgenden Formel A:

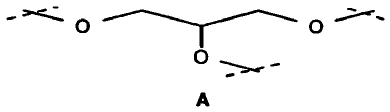

Die erfindungsgemäßen Umsetzungsprodukte IIIa und IIIb entstehen nicht nur bei einer direkten Umsetzung des erfindungsgemäßen Diols Ia oder des Chlorhydrins Ib mit 1-Halogen-2,3-epoxypropan, bevorzugt Epichlorhydrin oder 1-Hydroxy-2,3-epoxypropan, sondern können auch als Nebenprodukt bei der Herstellung des erfindungsgemäßen Diols Ia, des erfindungsgemäßen Chlorhydrins Ib, des erfindungsgemäßen Alkylglycidolcarbonats Ic oder des erfindungsgemäßen Epoxids Id ausgehend von 2-Propylheptanol, das mit 1-Hydroxy-2,3-epoxypropan oder 1-Halogen-2,3-epoxypropan, bevorzugt Epichlorhydrin, umgesetzt wird, gebildet werden.

Die verschiedenen Verbindungen der Formel I stellen unterschiedliche Vorstufen bei der Herstellung des erfindungsgemäßen Alkylglycidolcarbonats dar. Die vorliegende Erfindung betrifft neben den erfindungsgemäßen Verbindungen der Formel I Verfahren zur Herstellung der einzelnen Vorstufen, sowie Verfahren zur Herstellung des erfindungsgemäßen Alkylglycidolcarbonats der Formel Id.

Der erste Schritt bei der Herstellung des erfindungsgemäßen Alkylglycidolcarbonats der Formel Id ist die Herstellung einer Verbindung der Formel Ib, worin A eine OH-Gruppe und B ein Halogenatom, bevorzugt ein Cl-Atom, ist.

Somit ist ein weiterer Gegenstand der vorliegenden Erfindung ein Verfahren zur Herstellung von Verbindungen der allgemeinen Formel Ib 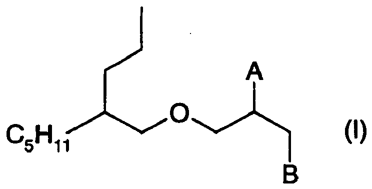 worin A eine OH-Gruppe und B ein Halogenatom, bevorzugt ein Cl-Atom, ist, und
C₅H₁₁ ein unverzeigter oder verzweigter C₅H₁₁-Alkylrest oder ein Gemisch aus unverzweigten und verzweigten C₅H₁₁-Alkylresten ist,
durch Umsetzung von 2-Propylheptanol mit 1-Halogen-2,3-epoxypropan, bevorzugt Epichlorhydrin, bevorzugt in Anwesenheit einer Säure, besonders bevorzugt in Anwesenheit von katalytischen Mengen einer Lewis-Säure.

Bevorzugte Reste C₅H₁₁ wurden bereits vorstehend genannt.

Geeignete Lewis-Säuren sind z.B. AlCl₃, BF₃ x Et₂O, BF₃, BF₃ x H₃PO₄, SbCl₄ x 2 H₂O und Hydrotalcit genauso wie Doppelmetallcyanid-Katalysatoren. Bevorzugt sind BF₃ x Et₂O, BF₃ und SbCl₄ x 2 H₂O. Besonders bevorzugt wird BF₃ x Et₂O eingesetzt.

Im allgemeinen werden 2-Propylheptanol und 1-Halogen-2,3-epoxypropan, bevorzugt Epichlorhydrin, in einem molaren Verhältnis von 1 zu 0,5 bis 1 zu 10, besonders bevorzugt 1 zu 0,75 bis 1 zu 5, ganz besonders bevorzugt ca. 1 : 1 eingesetzt.

Die als Katalysator eingesetzte Lewis-Säure wird im allgemeinen in einer Menge von 0,05 bis 5 Gew.-%, bevorzugt 0,05 bis 2 Gew.-%, besonders bevorzugt 0,1 bis 1 Gew.-%, bezogen auf die Masse des Gesamtansatzes, eingesetzt.

Die Reaktion kann in einem organischen Lösungsmittel z.B. Hexan, Toluol, Diethylether, tert-Butylmethylether, THF oder Dibutylether durchgeführt werden. Bevorzugt verwendet man kein Lösungsmittel.

Die Reaktionstemperatur beträgt im allgemeinen -20 °C bis 150 °C, bevorzugt -5 °C bis 120 °C, besonders bevorzugt 20 °C bis 80 °C. Der Reaktionsdruck beträgt im allgemeinen 1 bis 10 bar, bevorzugt 1 bis 6 bar, besonders bevorzugt Atmosphärendruck.

Die Reaktion kann nach dem Fachmann bekannten Methoden durchgeführt werden. In einer bevorzugten Ausführungsform wird 2-Propylheptanol gemeinsam mit der Lewis-Säure vorgelegt und auf die Reaktionstemperatur erwärmt. 1-Halogen-2,3-epoxypropan wird nach und nach zugegeben. Anschließend kann noch nachgerührt werden, zur Vervollständigung der Reaktion.

Eine Isolierung und Reinigung des Produkts, z.B. durch Destillation, ist möglich. Das Produkt kann jedoch auch ohne Aufreinigung in einer Folgestufe bei der Herstellung des erfindungsgemäßen Alkylglycidolcarbonats eingesetzt werden.

Die Reaktion kann in dem Fachmann bekannten Vorrichtungen durchgeführt werden.

Der zweite Schritt bei der Herstellung des erfindungsgemäßen Alkylglycidolcarbonats ist die Herstellung einer Verbindung der Formel Id, worin A und B gemeinsam einen Rest der Formel 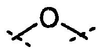 darstellen, d.h. Teil desselben Epoxidrings sind.

Somit ist ein weiterer Gegenstand der vorliegenden Erfindung ein Verfahren zur Herstellung einer Verbindung der allgemeinen Formel Id, 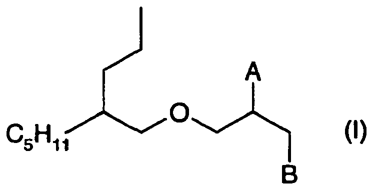 worin A und B gemeinsam einen Rest der Formel 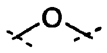 darstellen, d.h. Teil desselben Epoxidrings sind, durch Umsetzung einer Verbindung der Formel I, worin A eine OH-Gruppe und B ein Halogenatom ist, mit einer Base.

Bevorzugte Reste C₅H₁₁ wurden bereits vorstehend genannt.

Geeignete Basen sind z.B. Triethylamin, Dimethylcyclohexylamin, Na₂CO₃, NaOH, KOH, Na₃PO₄, Na₂HPO₄, Natriummethylat, Kaliumtertbutylat. Bevorzugt sind Triethylamin, Dimethylcyclohexylamin, NaOH und KOH. Besonders bevorzugt wird NaOH als wäßrige Lösung eingesetzt.

Die Base wird im allgemeinen in einer Menge von 0,5 bis 10 Äquivalenten, bevorzugt 0,8 bis 2 Äquivalenten, besonders bevorzugt 1,0 bis 1,5 Äquivalenten, bezogen auf die Verbindung der Formel Ib, worin A eine OH-Gruppe und B ein Halogenatom, bevorzugt ein Cl-Atom ist, eingesetzt.

Die Umsetzung wird im allgemeinen in einem zweiphasigen System, bei dem die Base in der wäßrigen Phase löslich ist, oder einphasig durchgeführt. Bevorzugt ist die Verwendung eines zweiphasigen Gemisches, bei dem die Base in Wasser gelöst vorliegt und Chlorhydrin bzw. Epoxid die organische Phase bilden. Nach einer besonders bevorzugten Variante wird das Reaktionsprodukt durch Phasentrennung von der wäßrigen Phase abgetrennt..

Die Reaktionstemperatur beträgt im allgemeinen 20 °C bis 150 °C, bevorzugt 40 °C bis 120 °C, besonders bevorzugt 50 °C bis 110 °C. Der Reaktionsdruck beträgt im allgemeinen 1 bis 10 bar, bevorzugt 1 bis 6 bar, besonders bevorzugt Atmosphärendruck.

Die Reaktion kann nach dem Fachmann bekannten Methoden durchgeführt werden. In einer bevorzugten Ausfiihrungsform wird die Verbindung der Formel Ib, worin A eine OH-Gruppe und B ein Halogenatom, bevorzugt ein Cl-Atom ist, vorgelegt und die Base, bevorzugt NaOH in Wasser, nach und nach zugegeben, wobei langsam auf die Reaktionstemperatur erwärmt wird. Die umgekehrte Zugabe des Chlorhydrins zu einer wäßrigen Lösung von NaOH in Wasser ist ebenfalls möglich und im Rahmen dieser Erfindung bevorzugt Nach Abschluß der Reaktion erfolgt eine Phasentrennung. Die organische Phase enthält dann das gewünschte Produkt. Eine Reinigung des Produkts, ist möglich. Das Produkt kann jedoch auch ohne Aufreinigung in einer Folgestufe bei der Herstellung des erfindungsgemäßen Alkylglycidolcarbonats eingesetzt werden.

Die Reaktion kann in dem Fachmann bekannten Vorrichtungen durchgeführt werden.

Der dritte Schritt bei der Herstellung des erfindungsgemäßen Alkylglycidolcarbonats ist die Herstellung einer Verbindung der Formel Ia, worin sowohl A als auch B eine OH-Gruppe sind.

Somit ist ein weiterer Gegenstand der vorliegenden Erfindung ein Verfahren zur Herstellung einer Verbindung der Formel Ia, worin sowohl A als auch B eine OH-Gruppe sind, durch Hydrolyse einer Verbindung der allgemeinen Formel Id, worin A und B gemeinsam einen Rest der Formel 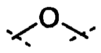 darstellen, d.h. Teil desselben Epoxidrings sind, in Anwesenheit eines Katalysators.

Bevorzugte Reste C₅H₁₁ wurden bereits vorstehend genannt.

Geeignete Katalysatoren sind z.B. Broensted Säuren, Broensted Basen, Übergangsmetallkomplexe und Lewis Säuren. Bevorzugt sind Broensted Säuren und Übergangsmetall-Komplexe. Besonders bevorzugt werden Übergangsmetall-Salen-Komplexe eingesetzt.

Im Fall von Broensted Säuren werden Säuren wie Salzsäure oder Schwefelsäure bevorzugt in wässriger Lösung eingesetzt. Epoxid (Id) und Diol (Ia) können in einem organischen Lösungsmittel gelöst oder bevorzugt ohne weiteres organisches Lösungsmittel umgesetzt werden. Die Säuren gibt man in 0.5 bis 10 Äquivalenten bezogen auf Epoxid (Ia) zu. Bevorzugt in 1 bis 3 Äquivalenten.

Als Übergangsmetall-Salen Komplexe sind N,N'-Bis(3,5-di-tert-butylsalicyliden)-1,2-cyclohexan-diaminocobalt-Verbindungen bevorzugt. Wenn diese verwendet werden, kann man die Umsetzung in einem organischen Lösungsmittel wie Toluol durchführen oder bevorzugt ohne Lösungsmittel arbeiten und 0,5 bis 20 Äquivalente, bevorzugt 0,8 bis 10, besonders bevorzugt 1,0 bis 3,0 Äquivalente Wasser zugeben. Der Katalysator wird in Mengen von 0,1 bis 10 mol% bevorzugt 0,3 bis 5 mol%, besonders bevorzugt 0,5 bis 3 mol% bezogen auf das Epoxid (Id) eingesetzt.

Die Reaktionstemperatur beträgt im allgemeinen 0 °C bis 200 °C, bevorzugt 20 bis 160 °C, besonders bevorzugt 20 bis 100 °C. Der Reaktionsdruck beträgt im allgemeinen 1 bis 10 bar, bevorzugt 1 bis 6 bar, besonders bevorzugt Atmosphärendruck.

Die Reaktion kann nach dem Fachmann bekannten Methoden durchgeführt werden. In einer bevorzugten Ausführungsform wird das Epoxid gemäß Formel Id zu dem eingesetzten Katalysator und Wasser gegeben. Anschließend kann noch nachgerührt werden, zur Vervollständigung der Reaktion.

Im allgemeinen kann das Produkt auch ohne weitere Reinigung ggf. nach Abtrennen des Lösungsmittels verwendet werden. Bevorzugt wird das Produkt durch Destillation des nach der Reaktion erhaltenen Reaktionsgemischs erhalten und kann in dieser Form in einer Folgestufe bei der Herstellung des erfindungsgemäßen Alkylglycidolcarbonats eingesetzt werden.

Die Reaktion kann in dem Fachmann bekannten Vorrichtungen durchgeführt werden.

Das gewünschte erfindungsgemäße Diol der Formel Ia kann des weiteren z.B. durch Umsetzung von 2-Propylheptanol mit Hydroxy-2,3-epoxypropan (Glycidol) erhalten werden.

Ein weiterer Gegenstand der vorliegenden Erfindung ist daher ein Verfahren zur Herstellung eines Diols gemäß der allgemeinen Formel Ia, worin A und B eine OH-Gruppe sind, durch Umsetzung von 2-Propylheptanol mit Hydroxy-2,3-epoxypropan (Glycidol).

Die gewünschten Alkylglycidolcarbonate können prinzipiell auf zwei wichtigen Wegen hergestellt werden. Einerseits durch Umsetzung von Diolen mit Phosgen (wie z. B. beschrieben in der WO 98 00418), andererseits durch Umsetzung von Epoxiden mit CO₂ unter Verwendung eines Katalysators (Paddock, Nguyen, J. Am. Chem. Soc. 2001, 123, 11498; Kisch, Millini, Wang, Chem. Ber. 1986, 119 (3), 1090; Baba, Nozaki, Matsuda, Bull. Chem. Soc. Jpn. 1987, 60 (4), 1552; Lermontov, Velikokhat'ko, Zavorin, Russ. Chem. Bull. 1998, 47 (7), 1405; Rokicki, Kuran, Pogorzelska-Marciniak, Monatshefte für Chemie 1984,115,205).

Bei der Phosgen-Umsetzung wird stets das Diol mit Phosgen unter Abspaltung von HCl umgesetzt. Das entstehende HCl wird durch Zugabe einer Base neutralisiert.

Bei der CO₂-Umsetzung wird das Epoxid unter erhöhtem Druck und erhöhter Temperatur mit Kohlendioxid umgesetzt. Verfügbare Katalysatoren für die Umsetzung sind z. B. Amine, Übergangsmetall-Salen-Komplexe, Zink-Salze oder Kombinationen von Zink-Salzen mit quartären Ammoniumsalzen.

Das in dem dritten Reaktionsschritt oder durch Umsetzung von 2-Propylheptanol mit Glycidol erhaltene Diol Ia kann somit zu dem gewünschten Alkylglycidolcarbonat umgesetzt werden. Es ist ebenfalls möglich, das in dem zweiten Reaktionsschritt erhaltene Epoxid der Formel Id zu dem gewünschten Alkylglycidolcarbonat umzusetzen.

Ein weiterer Gegenstand der vorliegenden Erfindung ist daher ein Verfahren zur Herstellung eines Alkylglycidolcarbonats der Formel Ic durch Umsetzung des Diols der Formel I mit Phosgen.

Diese Umsetzung erfolgt in einer bevorzugten Ausführungsform durch Zusammengeben einer gekühlten Lösung von Phosgen in einem aromatischen Lösungsmittel, bevorzugt Toluol, zu einer gekühlten Lösung des Diols der Formel Ia in einem aromatischen Lösungsmittel, bevorzugt ebenfalls Toluol, in Anwesenheit einer Base, bevorzugt eines Amins, besonders bevorzugt Triethylamin oder Dimethylcyclohexylamin, zur Neutralisation von bei der Umsetzung gebildeter HCl. Die Temperatur sollte während des Zusammengebens 0°C nicht übersteigen. Sie beträgt bevorzugt -5°C bis 0°C. Nach Erwärmung der Reaktionsmischung auf Raumtemperatur wird die Umsetzung für im allgemeinen 1 bis 20 Stunden, bevorzugt 12 bis 16 Stunden bei Raumtemperatur weitergeführt. Nach Beendigung der Umsetzung erfolgt eine Aufarbeitung und anschließende Reinigung des gewünschten Alkylglycidolcarbonats nach dem Fachmann bekannten Methoden. Die bevorzugt eingesetzte Aminbase kann gegebenenfalls als Hydrochlorid isoliert und dem Verfahren nach Freisetzen des Amins und gegebenenfalls Abtrennung von Wasser wieder zugeführt werden.

Phosgen wird im allgemeinen in einem 0 bis 50 %igem Überschuss, bevorzugt in einem 0 bis 15 %igem Überschuss im Verhältnis zu dem Diol der Formel Ia eingesetzt. Dabei bedeutet ein 0 %iger Überschuss, dass Phosgen und das Diol in äquimolaren Mengen eingesetzt werden. Die eingesetzte Base wird im allgemeinen in einem molaren Verhältnis zu Phosgen von im allgemeinen 2 zu 1 bis 4 zu 1, bevorzugt 2 zu 1 bis 2,5 zu 1 eingesetzt.

Ein weiterer Gegenstand der vorliegenden Erfindung ist ein Verfahren zur Herstellung des erfindungsgemäßen Alkylglycidolcarbonats der Formel Ic durch Umsetzung des Epoxids gemäß Formel Id mit CO₂ in Anwesenheit eines Katalysators.

Bei der Umsetzung mit CO₂ wird das Epoxid der Formel Id unter erhöhtem Druck von im allgemeinen 1 bis 50 bar, bevorzugt 1 bis 20 bar (sollten wir in der anderen Anmeldung, wenn noch möglich, auch auf 20 bar erhöhen) und erhöhter Temperatur von im allgemeinen 25 bis 150 °C, bevorzugt 40 bis 120 °C mit Kohlendioxid umgesetzt. Verfügbare Katalysatoren für die Umsetzung sind z. B. Amine, Übergangsmetall-Salen-Komplexe, Zink-Salze oder Kombinationen von Zink-Salzen mit quartären Ammoniumsalzen. Die anschließende Aufarbeitung und Reinigung des gewünschten Alkylglycidolcarbonats erfolgt nach dem Fachmann bekannten Methoden.

Ein weiterer Gegenstand der vorliegenden Erfindung ist ein Verfahren zur Herstellung des erfindungsgemäßen Alkylglycidolcarbonats der Formel Ic, umfassend alle oder mehrere der folgenden Schritte:
a) Umsetzung von 2-Propylheptanol mit 1-Halogen-2,3-epoxypropan, wobei eine Verbindung der Formel Ib 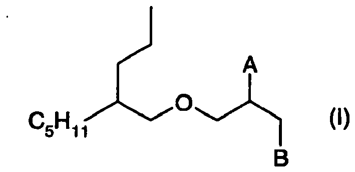 gebildet wird, worin A eine OH-Gruppe und B ein Halogenatom sind,
b) Umsetzung der in Schritt a) gebildeten Verbindung mit einer Base, wobei eine Verbindung der Formel Id gebildet wird, worin A und B gemeinsam einen Rest der Formel 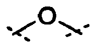 darstellen, d.h. Teil desselben Epoxidrings sind.
c) Hydrolyse der in Schritt b) gebildeten Verbindung, wobei ein Diol der Formel Ia gebildet wird,
d) Umsetzung der in Schritt d) gebildeten Verbindung mit Phosgen, wobei die gewünschte Verbindung (Alkylglycidolcarbonat, Struktur Ic) gebildet wird; oder
e) Umsetzung der in Schritt b) gebildeten Verbindung mit CO₂ in Anwesenheit eines Katalysators, wobei die gewünschte Verbindung (Alkylglycidolcarbonat, Struktur Ic) gebildet wird.

Bevorzugte Ausführungsformen der einzelnen Reaktionsschritte sind bereits vorstehend beschrieben.

Des weiteren sind die erfindungsgemäßen Alkylglycidolcarbonate Ic durch ein Verfahren erhältlich, umfassend die folgenden Schritte:
a) Umsetzung von 2-Propylheptanol mit Glycidol, wobei ein Diol der Formel Ia gebildet wird,
b) Umsetzung der in Schritt b) gebildeten Verbindung mit Phosgen, wobei die gewünschte Verbindung (Alkylglycidolcarbonat, Struktur Ic) gebildet wird.

Bevorzugte Ausführungsformen der einzelnen Reaktionsschritte sind bereits vorstehend beschrieben.

In dem folgenden Schema sind die erfindungsgemäßen Herstellungswege zur Herstellung des Allcylglycidolcarbonats der Formel Ic ausgehend von 2-Propylheptanol, sowie die bei der Herstellung entstehenden erfindungsgemäßen Vorstufen zusammengefasst:

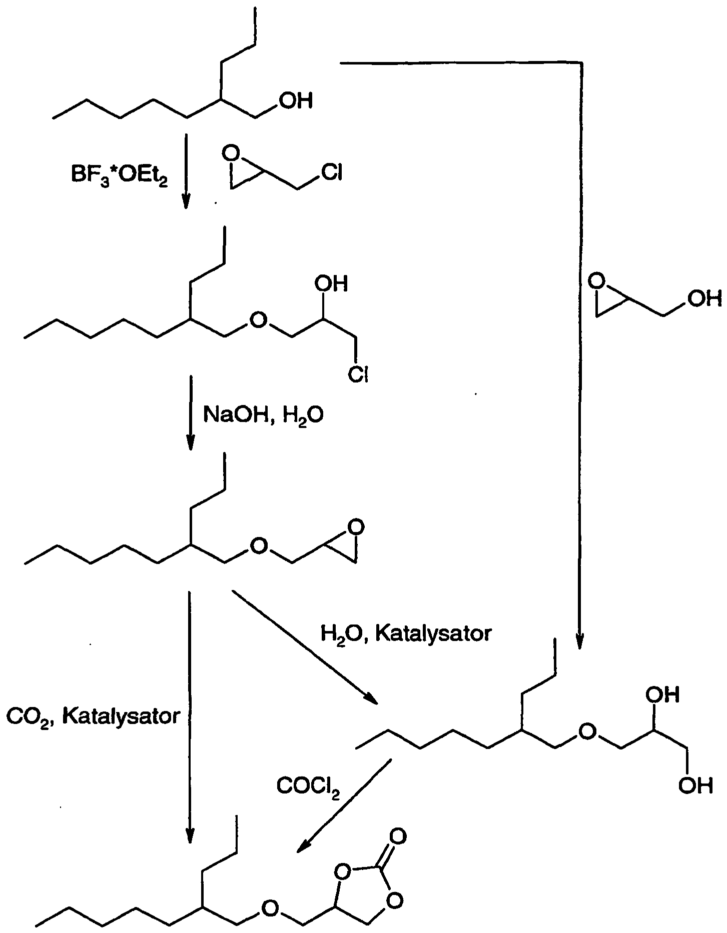

Des weiteren ist Gegenstand der Erfindung ein Verfahren zur Herstellung eines Umsetzungsproduktes IIIa gemäß der vorliegenden Anmeldung, durch Umsetzung eines Moläquivalents des Diols der Formel Ia mit 1 bis 10 Moläquivalenten, bevorzugt 1 bis 4 Moläquivalenten, besonders bevorzugt 1 bis 1,5 Moläquivalenten 1-Hydroxy-2,3-epoxypropan. Die erfindungsgemäßen Umsetzungsprodukte sind auch auf anderen Wegen erhältlich.

Des weiteren ist Gegenstand der Erfindung ein Verfahren zur Herstellung eines Umsetzungsproduktes IIIb gemäß der vorliegenden Anmeldung, durch Umsetzung eines Moläquivalents des Halohydrins (bevorzugt Chlorhydrins) der Formel Ib mit 1 bis 10 Moläquivalenten, bevorzugt 1 bis 4 Moläquivalenten, besonders bevorzugt 1 bis 1,5 Moläquivalenten 1-Halogen-2,3-epoxypropan, bevorzugt Epichlorhydrin. Die erfindungsgemäßen Umsetzungsprodukte sind auch auf anderen Wegen erhältlich.

Ein weiterer Gegenstand der Erfindung ist die Verwendung des Alkylglycidolcarbonats der allgemeinen Formel Ic als Co-Tensid.

Die erfindungsgemäßen, als Co-Tenside zu verwendenden Alkylglycidolcarbonate der Formel Ic eigenen sich zum Einsatz in industriellen, institutionellen oder Haushaltswaschmitteln und -reinigern sowie im sogenannten Body-Care-Sektor, also Körperreinigungs- und -pflegemitteln.

Weitere Anwendungen sind:
- Feuchthaltemittel, insbesondere für die Druckindustrie.
- Kosmetische, pharmazeutische und Pflanzenschutzformulierungen. Geeignete Pflanzenschutzformulierungen sind beispielsweise in der EP-A-0 050 228 beschrieben. Es können für Pflanzenschutzmittel übliche weitere Inhaltsstoffe vorliegen.
- Lacke, Beschichtungsmittel, Farben, Pigmentpräparationen sowie Klebstoffe in der Lack- und Folienindustrie.
- Lederentfettungsmittel.
- Formulierungen für die Textilindustrie wie Egalisiermittel oder Formulierungen zur Garnreinigung.
- Faserverarbeitung und Hilfsmittel für die Papier- und Zellstoffindustrie.
- Metallverarbeitung wie Metallveredelung und Galvanobereich.
- Lebensmittelindustrie.
- Wasserbehandlung und Trinkwassergewinnung.
- Fermentation.
- Mineralverarbeitung und Staubkontrolle.
- Bauhilfsmittel.
- Emulsionspolymerisation und Herstellung von Dispersionen.
- Kühl- und Schmiermittel.

Die Waschmittel liegen in fester, flüssiger, gelförmiger oder pastenförmige Form vor. Zu den in fester Form vorliegenden Materialien gehören Pulver und Kompaktate, beispielsweise Granulate und Formkörper wie Tabletten.

Die Waschmittel enthalten 0,1 bis 40 Gew-%, insbesondere 0,5 bis 30 Gew-%, ganz besonders 1 bis 20 Gew-%, bezogen auf die Gesamtmenge der Formulierung, mindestens einer Substanz der Formeln I und/oder II. Weitere Bestandteile sind nachfolgend aufgeführt.

Waschmittelformulierungen enthalten üblicherweise Inhaltsstoffe wie Tenside, Gerüst-, Duft- und Farbstoffe, Komplexbildner, Polymere und andere Inhaltsstoffe. Typische Formulierungen sind beispielsweise in WO 01/32820 beschrieben. Weitere für unterschiedliche Anwendungen geeignete Inhaltsstoffe sind in EP-A-0 620 270, WO 95/27034, EP-A-0 681 865, EP-A-0 616 026, EP-A-0 616 028, DE-A-42 37 178 und US 5,340,495 beispielhaft beschrieben.

Waschmittel im Sinne dieser Erfindung dienen in der Regel zum Waschen von mehr oder weniger flexiblen Materialien, vorzugsweise solchen, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen.

Es kann sich um Rohfasern oder um Fasern in beliebigen Verarbeitungsstadien handeln und es können natürliche Eiweiß- oder Zellulosefasern wie Wolle, Seide, Baumwolle, Sisal, Hanf, Kokosfasern oder Synthesefasern wie beispielsweise Polyester-, Polyamid- oder Polyacrylnitrilfasern sein.

Waschmittel enthaltend die erfindungsgemässen Co-Tenside können auch zur Reinigung von faserhaltigen Materialien, wie z. B. rückenbeschichteten Teppichen mit geschnittenem oder ungeschnittenem Flor dienen.

Die Zusammensetzungen der Waschmittel werden vorzugsweise den verschiedenen Zwecken angepasst, wie es dem Fachmann aus dem Stand der Technik geläufig ist. Hierzu können den Waschmitteln alle zweckentsprechenden aus dem Stand der Technik bekannten Hilfs- und Zusatzstoffe zugefügt werden.

In Waschmitteln können neben den erfindungsgemäßen Co-Tensiden beispielsweise vorliegen:
- Builder und Cobuilder, wie Polyphosphate, Zeolithe, Polycarboxylate, Phosphonate oder Komplexbildner
- ionische Tenside, wie Alkoholsulfate/-ethersulfate, Alkylbenzolsulfonate, α-Olefinsulfonate und andere Alkoholsulfate/-ethersulfate
- nichtionische Tenside, Alkoholalkoxylate wie Alkylaminalkoxylate, Alkylpolyglucoside
- optische Aufheller
- Farbübertragungsinhibitoren, wie Polyvinylpyrrolidon der Molmassen 8.000 bis 70.000, Vinylimidazol/Vinylpyrrolidon-Copolymere mit einem Molverhältnis der Monomeren von 1:10 bis 2:1 und Molmassen von 8.000 bis 70.000 sowie Poly-4-vinylpyridin-N-oxide mit Molmassen von 8.000 bis 70.000.
- Stellmittel, wie Natriumsulfat oder Magnesiumsulfat
- Soil Release-Mittel
- Inkrustationsinhibitoren
- Bleichsysteme, enthaltend Bleichmittel, wie Perborat, Percarbonat und Bleichaktivatoren, wie Tetraacetylethylendiamin sowie Bleichstabilisatoren
- Parfum/-öle
- Schaumdämpfer, wie Silikonöle
- Enzyme, wie Amylasen, Lipasen, Cellulasen, Proteasen
- Alkalispender, wie lösliche Alkalisilikate, z. B. Pentanatriummethasilikat, Nariumcarbonat.

In flüssigen Waschmitteln können beispielsweise zusätzlich Lösungsmittel, wie Ethanol, Isopropanol, 1,2-Propylenglycol, Butylglycol usw. eingesetzt werden.

In tablettenförmigen Waschmitteln können zusätzlich Tablettierhilfsmittel, wie Polyethylenglycole mit Molmassen von mehr als 1000 g/mol, Polymerdispersionen sowie Tablettensprengmittel, wie Cellulosederivate, vernetztes Polyvinylpyrrolidon, vernetzte Polyacrylate oder Kombinationen aus Säuren, wie Zitronensäure und Natriumbicarbonat eingesetzt werden. Eine detailliertere Aufführung möglicher Inhaltsstoffe wird nachfolgend gegeben.

In manchen Fällen kann es zweckmäßig sein, die erfindungsgemäß eingesetzten Co-Tenside mit anderen Co-Tensiden oder mit amphoteren Tensiden, wie z. B. Alkylaminoxiden, oder Betainen zu kombinieren.

Eine andere Klasse nichtionischer Tenside sind Alkylpolyglucoside mit 6 bis 22, vorzugsweise 10 bis 18 Kohlenstoffatomen in der Alkylkette. Diese Verbindungen enthalten meist 1 bis 20, vorzugsweise 1,1 bis 5 Glucosideinheiten.

Eine andere Klasse nichtionischer Tenside sind N-Alkylglucamide der allgemeinen Strukturen 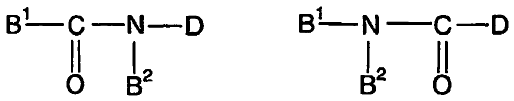 wobei B¹ ein C₆- bis C₂₂-Alkyl, B² Wasserstoff oder C₁- bis C₄-Alkyl und D ein Polyhydroxyalkyl-Rest mit 5 bis 12 C-Atomen und mindestens 3 Hydroxygruppen ist. Vorzugsweise steht B¹ für C₁₀- bis C₁₈-Alkyl, B² für CH₃ und D für einen C₅- oder C₆-Rest. Beispielsweise erhält man derartige Verbindungen durch die Acylierung von reduzierend aminierten Zuckern mit Säurechloriden von C₁₀- bis C₁₈-Carbonsäuren.

Weitere in Betracht kommende nichtionische Tenside sind die aus der WO-A 95/11225 bekannten endgruppenverschlossenen Fettsäureamidalkoxylate der allgemeinen Formel

R¹-CO-NH-(CH₂)_{y}-O-(A¹O)ₓ-R²

in der
- R¹: einen C₅- bis C₂₁-Alkyl- oder Alkenylrest bezeichnet,
- R²: eine C₁- bis C₄-Alkylgruppe bedeutet,
- A¹: für C₂- bis C₄-Alkylen steht,
- y: die Zahl 2 oder 3 bezeichnet und
- x: einen Wert von 1 bis 6 hat.

Beispiele für solche Verbindungen sind die Umsetzungsprodukte von n-Butyltriglykolamin der Formel H₂N-(CH₂-CH₂-O)₃-C₄H₉ mit Dodecansäuremethylester oder die Reaktionsprodukte von Ethyltetraglykolamin der Formel H₂N-(CH₂-CH₂-O)₄-C₂H₅ mit einem handelsüblichen Gemisch von gesättigten C₈- bis C₁₈-Fettsäuremethylestern.

Weiterhin eignen sich als nichtionische Tenside noch Blockcopolymere aus Ethylenoxid, Propylenoxid und/oder Butylenoxid (Pluronic®- und Tetronic®-Marken der BASF), Polyhydroxy- oder Polyalkoxyfettsäurederivate wie Polyhydroxyfettsäureamide, N-Alkoxy- oder N-Aryloxypolyhydroxyfettsäureamide, Fettsäureamidethoxylate, insbesondere endgruppenverschlossene, sowie Fettsäurealkanolamidalkoxylate.

Die zusätzlichen nichtionischen Tenside ("Niotenside") liegen in den Waschmitteln enthaltend die erfindungsgemäss verwendetenen Co-Tenside vorzugsweise in einer Menge von 0,01 bis 30 Gew.-%, insbesondere 0,1 bis 25 Gew.-%, vor allem 0,5 bis 20 Gew.-%, vor.

Man kann zusätzlich einzelne nichtionische Tenside oder eine Kombination unterschiedlicher Niotenside einsetzen. Es können nichtionische Tenside aus nur einer Klasse zum Einsatz gelangen, insbesondere nur alkoxylierte C₈- bis C₂₂-Alkohole, man kann aber auch Tensidmischungen aus verschiedenen Klassen verwenden.

Geeignete anionische Tenside sind beispielsweise Fettalkoholsulfate von Fettalkoholen mit 8 bis 22, vorzugsweise 10 bis 18 Kohlenstoffatomen, C₁₂-C₁₈-Alkoholsulfate, Laurylsulfat, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, dass man zunächst einen C₈- bis C₂₂-, vorzugsweise einen C₁₀- bis C₁₈-Alkohol z. B. einen Fettalkohol, alkoxyliert und das Alkoxylierungsprodukt anschließend sulfatiert. Für die Alkoxylierung verwendet man vorzugsweise Ethylenoxid, wobei man pro Mol Alkohol 1 bis 50, vorzugsweise 1 bis 20 Mol Ethylenoxid einsetzt die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid oder Ethylenoxid und Propylenoxid und Butylenoxid enthalten. Die alkoxylierten C₈- bis C₂₂-Alkohole können die Ethylenoxid-, Propylenoxid- und Butylenoxideinheiten in Form von Blöcken oder in statistischer Verteilung enthalten. Je nach Art des Alkoxylierungskatalysators kann man Alkylethersulfate mit breiter oder enger Alkylenoxid-Homologen-Verteilung erhalten.

Weitere geeignete anionische Tenside sind Alkansulfonate wie C₈- bis C₂₄-, vorzugsweise C₁₀- bis C₁₈-Alkansulfonate sowie Seifen wie beispielsweise die Na- und K-Salze von gesättigten und/oder ungesättigten C₈₋ bis C₂₄-Carbonsäuren.

Weitere geeignete anionische Tenside sind lineare C₈- bis C₂₀-Alkylbenzolsulfonate ("LAS"), vorzugsweise lineare C₉- bis C₁₃-Alkylbenzolsulfonate und -Alkyltoluolsulfonate.

Weiterhin eignen sich als anionische Tenside noch C₈- bis C₂₄-Olefinsulfonate und -disulfonate, welche auch Gemische aus Alken- und Hydroxyalkansulfonaten bzw. -disulfonate darstellen können, Alkylestersulfonate, sulfonierte Polycarbonsäuren, Alkylglycerinsulfonate, Fettsäureglycerinestersulfonate, Alkylphenolpolyglykolethersulfate, Paraffinsulfonate mit ca. 20 bis ca. 50 C-Atomen (basierend auf aus natürlichen Quellen gewonnenem Paraffin oder Paraffingemischen), Alkylphosphate, Acylisethionate, Acyltaurate, Acylmethyltaurate, Alkylbernsteinsäuren, Alkenylbernsteinsäuren oder deren Halbester oder Halbamide, Alkylsulfobemsteinsäuren oder deren Amide, Mono- und Diester von Sulfobernsteinsäuren, Acylsarkosinate, sulfatierte Alkylpolyglucoside, Alkylpolyglykolcarboxylate sowie Hydroxyalkylsarkosinate.

Die anionischen Tenside werden dem Waschmittel vorzugsweise in Form von Salzen zugegeben. Geeignete Kationen in diesen Salzen sind Alkalimetallionen wie Natrium, Kalium und Lithium und Ammoniumsalze wie z. B. Hydroxyethylammonium-, Di(hydroxyethyl)ammonium- und Tri(hydroxyethyl)ammoniumsalze.

Die anionischen Tenside liegen in den Waschmitteln enthaltend die erfindungsgemässen Co-Tenside vorzugsweise in einer Menge von bis zu 30 Gew.-%, beispielsweise von 0,1 bis 30 Gew.-%, vor allem 1 bis 25 Gew.%, insbesondere 3 bis 10 Gew.-% vor. Werden C₉- bis C₂₀-linear-Alkyl-benzolsulfonate (LAS) mitverwendet, kommen diese üblicherweise in einer Menge bis zu 15 Gew.-%, insbesondere bis zu 10 Gew.-%, zum Einsatz.

Man kann einzelne anionische Tenside oder eine Kombination unterschiedlicher Aniontenside einsetzen. Es können anionische Tenside aus nur einer Klasse zum Einsatz gelangen, beispielsweise nur Fettalkoholsulfate oder nur Alkylbenzolsulfonate, man kann aber auch Tensidmischungen aus verschiedenen Klassen verwenden, z. B. eine Mischung aus Fettalkoholsulfaten und Alkylbenzolsulfonaten.

Ferner können Tensidgemische enthaltend die erfindungsgemäss einzusetzenden Co-Tenside mit kationischen Tensiden, üblicherweise in einer Menge bis 25 Gew.-%, vorzugsweise 1 bis 15 Gew.-%, beispielsweise C₈-bis C₁₆-Dialkyldimethylammoniumsalzen, Dialkoxydimethyl-ammoniumsalzen oder Imidazoliniumsalzen mit langkettigem Alkylrest; und/oder mit amphoteren Tensiden, üblicherweise in einer Menge bis 15 Gew.-%, vorzugsweise 1 bis 10 Gew.-%, beispielsweise Derivaten von sekundären oder tertiären Aminen wie z. B. C₆-C₁₈-Alkylbetainen oder C₆-C₁₅-Alkylsulfobetainen oder Alkylamidobetainen oder Aminoxiden wie Alkyldimethylaminoxiden kombiniert werden. Ferner können kationische Tenside eingesetzt werden, wie sie in der WO 99/19435 beschrieben sind.

In der Regel werden Gemische enthaltend die erfindungsgemäss einzusetzenden Co-Tenside mit Buildern (Sequestrierungsmitteln) wie z. B. Polyphosphaten, Polycarboxilaten, Phosphonaten, Komplexbildnern, z. B. Methylglycindiessigsäure und deren Salze, Nitrilotriessigsäure und deren Salze, Ethylendiamintetraessigsäure und deren Salze sowie gegebenenfalls mit Co-Buildern kombiniert.

Einzelne zur Kombination mit Gemischen enthaltend die erfindungsgemäss einzusetzenden Co-Tenside gut geeignete Buildersubstanzen seien im Folgenden aufgezählt:

Geeignete anorganische Builder sind vor allem kristalline oder amorphe Alumosilicate mit ionenaustauschenden Eigenschaften wie insbesondere Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise beschrieben in der US-A-4604224.

Als Builder geeignete kristalline Silicate sind beispielsweise Disilicate oder Schichtsilicate, z. B. δ-Na₂SiO₅ oder ß-Na₂Si₂O₅. Die Silicate können in Form ihrer Alkalimetall-, Erdalkalimetall- oder Ammoniumsalze eingesetzt werden, vorzugsweise als Na-, Li- und Mg-Silicate.Amorphe Silicate wie beispielsweise Natriummetasilicat, welches eine polymere Struktur aufweist, oder amorphes Disilicat sind ebenfalls verwendbar.

Geeignete anorganische Buildersubstanzen auf Carbonat-Basis sind Carbonate und Hydrogencarbonate. Diese können in Form ihrer Akalimetall-, Erdalkalimetall- oder Ammoniumsalze eingesetzt werden. Vorzugsweise werden Na-, Li- und Mg-Carbonate bzw. - Hydrogencarbonate, insbesondere Natriumcarbonat und/oder Natriumhydrogencarbonat, eingesetzt.

Übliche, als anorganische Builder eingesetzte Phosphate sind Alkaliorthophosphate,und/oder -Polyphosphate wie z. B. Pentanatriumtriphosphat. Die genannten Builder-Komponenten können einzeln oder in Mischungen untereinander eingesetzt werden.

Ferner ist es in vielen Fällen zweckmäßig den Waschmitteln enthaltend die erfindungsgemäss einzusetzenden Co-Tenside Co-Builder zuzufügen. Beispiele für geeignete Substanzen sind im Folgenden aufgelistet:

In einer bevorzugten Ausführungsform enthalten die Waschmittel enthaltend die erfindungsgemäss einzusetzenden Co-Tenside zusätzlich zu den anorganischen Buildern 0,05 bis 20 Gew.-%, insbesondere 1 bis 10 Gew.-% organische Cobuilder in Form von niedermolekularen, oligomeren oder polymeren Carbonsäuren, insbesondere Polycarbonsäuren, oder Phosphonsäuren oder deren Salzen, insbesondere Na- oder K-salzen.

Als organische Cobuilder geeignete niedermolekulare Carbonsäuren oder Phosphonsäuren sind beispielsweise:
Phosphonsäuren wie z. B. 1-Hydroxyethan-1,1-diphosphonsäure, Amino-tris(methylenphosphonsäure), Ethylendiamin-tetra(methylenphosphonsäure), Hexamethylendiamin-tetra(methylenphosphonsäure) und Diethylentriamin-penta(methylenphosphonsäure);
C₄-bis C₂₀-Di-, -Tri- und -Tetracarbonsäuren wie z. B. Bernsteinsäure, Propantricarbonsäure, Butantetracarbonsäure, Cyclopentantetracarbonsäure und Alkyl- und Alkenylbernsteinsäuren mit C₂- bis C₁₆-Alkyl- bzw. -Alkenyl-Resten;
C₄- bis C₂₀-Hydroxycarbonsäuren wie z. B. Äpfelsäure, Weinsäure, Gluconsäure, Glutarsäure, Citronensäure, Lactobionsäure und Saccharosemono-, di- und tricarbonsäure;
Aminopolycarbonsäuren wie z. B. Nitrilotriessigsäure, ß-Alanindiessigsäure, Ethylendiamintetraessigsäure, Serindiessigsäure, Isoserindiessigsäure, Alkylethylendiamintriacetate, N,N-bis(Carboxymethyl)glutaminsäure, Ethylendiamindibernsteinsäure und N-(2-Hydroxyethyl)iminodiessigsäure, Methyl- und Ethylglycindiessigsäure.

Als organische Cobuilder geeignete oligomere oder polymere Carbonsäuren sind beispielsweise:
Oligomaleinsäuren, wie sie beispielsweise in EP-A 451508 und EP-A 396303 beschrieben sind;
Co- und Terpolymere ungesättigter C₄- bis C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können.

Als ungesättigte C₄- bis C₈-Dicarbonsäuren sind hierbei beispielsweise Maleinsäure, Fumarsäure, Itaconsäure und Citraconsäure geeignet. Bevorzugt wird Maleinsäure.

Die Gruppe (i) umfasst monoethylenisch ungesättigte C₃-C₈-Monocarbonsäuren wie z. B. Acrylsäure, Methacrylsäure, Crotonsäure und Vinylessigsäure. Bevorzugt werden aus der Gruppe (i) Acrylsäure und Methacrylsäure eingesetzt.

Die Gruppe (ii) umfasst monoethylenisch ungesättigte C₂- bis C₂₂-Olefine, Vinylalkylether mit C₁- bis C₈-Alkylgruppen, Styrol, Vinylester von C₁- bis C₈-Carbonsäuren, (Meth)acrylamid und Vinylpyrrolidon. Bevorzugt werden aus der Gruppe (ii) C₂- bis C₆-Olefine, Vinylalkylether mit C₁- bis C₄-Alkylgruppen, Vinylacetat und Vinylpropionat eingesetzt.

Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt.

Die Gruppe (iii) umfasst (Meth)acrylester von C₁- bis C₈-Alkoholen, (Meth)acrylnitril, (Meth)acrylamide von C₁- bis C₈-Aminen, N-Vinylformamid und N-Vinylimidazol.

Als organische Cobuilder eignen sich auch Homopolymere der monoethylenisch ungesättigten C₃-C₈-Monocarbonsäuren wie z. B. Acrylsäure, Methacrylsäure, Crotonsäure und Vinylessigsäure, insbesondere der Acrylsäure und Methacrylsäure,

Copolymere von Dicarbonsäuren, wie z. B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000;

Terpolymere aus Maleinsäure, Acrylsäure und einem Vinylester einer C₁-C₃-Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann;

Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls als organische Cobuilder.

Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden.

Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii).

Als Pfropfgrundlage sind abgebaute Polysaccharide wie z. B. sauer oder enzymatisch abgebaute Stärken, Inuline oder Zellulose, Eiweißhydrolysate und reduzierte (hydrierte oder hydrierend aminierte) abgebaute Polysaccharide wie z. B. Mannit, Sorbit, Aminosorbit und N-Alkylglucamin geeignet sowie auch Polyalkylenglycole mit Molmassen mit bis zu M_{w} = 5000 wie z. B. Polyethylenglycole, Ethylenoxid/Propylenoxid- bzw. Ethylenoxid/Butylenoxid bzw. Ethylenoxid/Propylenoxid/Butylenoxid-Blockcopolymere und alkoxylierte ein- oder mehrwertige C₁- bis C₂₂-Alkohole.(vgl. US-A-5756456)

Als organische Cobuilder geeignete Polyglyoxylsäuren sind beispielsweise beschrieben in EP-B-001004, US-A-5399286, DE-A-4106355 und EP-A-656914. Die Endgruppen der Polyglyoxylsäuren können unterschiedliche Strukturen aufweisen.

Als organische Cobuilder geeignete Polyamidocarbonsäuren und modifizierte Polyamidocarbonsäuren sind beispielsweise bekannt aus EP-A-454126, EP-B-511037, WO-A-94/01486 und EP-A-581452.

Als organische Cobuilder verwendet man insbesondere auch Polyasparaginsäuren oder Cokondensate der Asparaginsäure mit weiteren Aminosäuren, C₄- bis C₂₅-Mono- oder - Dicarbonsäuren und/oder C₄- bis C₂₅-Mono- oder -Diaminen. Besonders bevorzugt werden in phosphorhaltigen Säuren hergestellte, mit C₆- bis C₂₂-Mono- oder -Dicarbonsäuren bzw. mit C₆- bis C₂₂-Mono- oder -Diaminen modifizierte Polyasparaginsäuren eingesetzt.

Als organische Cobuilder eignen sich weiterhin Iminodibernsteinsäure, Oxydibemsteinsäure, Aminopolycarboxylate, Alkylpolyaminocarboxylate, Aminopolyalkylenphosphonate, Polyglutamate, hydrophob modifizierte Citronensäure wie z. B. Agaricinsäure, Poly-α-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Cobuilder verwendet werden.

Weitere geeignete (Co)builder sind in WO 99/19435 beschrieben.

In einer weiteren bevorzugten Ausführungsform enthalten die Waschmittel enthaltend die erfindungsgemäss einzusetzenden Co-Tenside zusätzlich, insbesondere zusätzlich zu den anorganischen Bildern, den anionischen Tensiden und/oder den nichtionischen Tensiden, 0,5 bis 20 Gew.-%, insbesondere 1 bis 10 Gew.-%, Glycin-N,N-diessigsäure-Derivate, wie sie in der WO 97/19159 beschrieben sind.

Häufig ist es auch zweckmäßig, den Waschmitteln enthaltend die erfindungsgemäss einzusetzenden Co-Tenside Bleichsysteme, bestehend aus Bleichmitteln, wie z. B. Perborat, Percarbonat und gegebenenfalls Bleichaktivatoren, wie z. B. Tetraacetylethylendiamin, + Bleichstabilisatoren sowie gegebenenfalls Bleichkatalysatoren zuzusetzen.

In diesen Fällen enthalten die Waschmittel enthaltend die erfindungsgemäss einzusetzenden Co-Tenside zusätzlich 0,5 bis 30 Gew.-%, insbesondere 5 bis 27 Gew.-%, vor allem 10 bis 23 Gew.-% Bleichmittel in Form von Percarbonsäuren, z. B. Diperoxododecandicarbonsäure, Phthalimidopercapronsäure oder Monoperoxophthalsäure oder - terephthalsäure, Addukten von Wasserstoffperoxid an anorganische Salze, z. B. Natriumperborat-Monohydrat, Natriumperborat-Tetrahydrat, Natriumcarbonat-Perhydrat oder Natriumphosphat-Perhydrat, Addukten von Wasserstoffperoxid an organische Verbindungen, z. B. Hamstoff-Perhydrat, oder von anorganischen Peroxosalzen, z. B. Alkalimetallpersulfaten, oder -peroxodisulfaten, gegebenenfalls in Kombination mit 0 bis 15 Gew.-%, vorzugsweise 0,1 bis 15 Gew.-%, insbesondere 0,5 bis 8 Gew.-%, Bleichaktivatoren.

Als Bleichaktivatoren eignen sich:
- polyacylierte Zucker, z. B. Pentaacetylglucose;
- Acyloxybenzolsulfonsäuren und deren Alkali- und Erdalkaiimetallsalze, z. B. Natrium-p-nonanoyloxybenzolsulfonat oder Natrium-p-benzoyloxybenzolsulfonat;
- N,N-diacylierte und N,N,N',N'-tetraacylierte Amine, z. B. N,N,N',N'-Tetraacetylmethylendiamin und -ethylendiamin (TAED), N,N-Diacetylanilin, N,N-Diacetyl-p-toluidin oder 1,3-diacylierte Hydantoine wie 1,3-Diacetyl-5,5-dimethylhydantoin;
- N-Alkyl-N-sulfonylcarbonamide, z. B. N-Methyl-N-mesylacetamid oder N-Methyl-N-mesylbenzamid;
- N-acylierte cyclische Hydrazide, acylierte Triazole oder Urazole, z. B. Monoacetylmaleinsäurehydrazid;
- O,N,N-trisubstituierte Hydroxylamine, z. B. O-Benzoyl-N,N-succinylhydroxylamin, O-Acetyl-N,N-succinylhydroxylamin oder O,N,N-Triacetylhydroxylamin;
- N,N`-Diacylsulfurylamide, z. B. N,N`-Dimethyl-N,N`-diacetylsulfurylamid oder N,N'-Diethyl-N,N'-dipropionyisulfurylamid;
- acylierte Lactame wie beispielsweise Acetylcaprolactam, Octanoylcaprolactam, Benzoylcaprolactam oder Carbonylbiscaprolactam;
- Anthranilderivate wie z. B. 2-Methylanthranil oder 2-Phenylanthranil;
- Triacylcyanurate, z. B. Triacetylcyanurat oder Tribenzoylcyanurat;
- Oximester und Bisoximester wie z. B. O-Acetylacetonoxim oder Bisisopropyliminocarbonat;
- Carbonsäureanhydride, z. B. Essigsäureanhydrid, Benzoesäureanhydrid, m-Chlorbenzoesäureanhydrid oder Phthalsäureanhydrid;
- Enolester wie z. B. Isopropenylacetat;
- 1,3-Diacyl-4,5-diacyloxy-imidazoline, z. B. 1,3-Diacetyl-4,5-diacetoxyimidazolin;
- Tetraacetylglycoluril und Tetrapropionylglycoluril; diacylierte 2,5-Diketopiperazine, z. B. 1,4-Diacetyl-2,5-diketopiperazin;
- ammoniumsubstituierte Nitrile wie z. B. N-Methylmorpholiniumacetonitrilmethylsulfat;
- Acylierungsprodukte von Propylendiharnstoff und 2,2-Dimethyl-propylendiharnstoff, z. B. Tetraacetylpropylendiharnstoff;
- α-Acyloxypolyacylmalonamide, z. B. α-Acetoxy-N,N'-diacetylmalonamid;
- Diacyl-dioxohexahydro-1,3,5-triazine, z. B. 1,5-Diacetyl-2,4-dioxohexahydro-1,3,5-triazin;
- Benz-(4H)1,3-oxazin-4-one mit Alkylresten, z. B. Methyl, oder aromatischen Resten z. B. Phenyl, in der 2-Position;
- kationische Nitrile, wie in DE-A-101 48 577 beschrieben.

Das beschriebene Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise beschrieben sind in US-A 5 360 569 und EP-A 453 003. Besonders wirksame Bleichkatalysatoren sind Mangankomplexe, die beispielsweise in der WO-A 94/21777 beschrieben sind. Solche Verbindungen werden im Falle ihres Einsatzes in den Waschmitteln höchstens in Mengen bis 1,5 Gew.-%, insbesondere bis 0,5 % Gew.-%, im Falle von sehr aktiven Mangankomplexen in Mengen bis zu 0,1 Gew.-%, eingearbeitet. Weitere geeignete Bleichkatalysatoren sind in WO 99/19435 beschrieben.

Weitere einsetzbare Bleichsysteme auf Basis von Arylimidoperalkansäuren sind in EP-A-0 325 288 und EP-A-0 490 409 beschrieben.

### Bleichstabilisator

Dabei handelt es sich um Additive, die Schwermetallspuren absorbieren, binden oder komplexieren können. Beispiele für erfindungsgemäß verwendbare Zusätze mit bleichstabilisierender Wirkung sind polyanionische Verbindungen wie Polyphosphate, Polycarboxylate, Polyhydroxypolycarboxylate, lösliche Silikate als vollständig oder teilweise neutralisierte Alkali- oder Erdalkalisalze, insbesondere als neutrale Na- oder Mg-Salze, die relativ schwache Bleichstabilisatoren sind. Starke erfindungsgemäß verwendbare Bleichstabilisatoren sind beispielsweise Komplexbildner, wie Ethylendiamintetraacetat (EDTA), Nitrilotriessigsäure (NTA), Methylglycindiessigsäure (MGDA), ß-Alanindiessigsäure (ADA), Ethylendiamin-N,N'-diesuccinat (EDDS) und Phosphonate wie Ethylendiamintetramethylenphosphonat, Diethylentriaminpentamethylenphosphonat oder Hydroxyethyliden-1,1-diphosphonsäure in Form der Säuren oder als teilweise oder vollständig neutralisierte Alkalimetallsalze. Vorzugsweise werden die Komplexbildner in Form ihrer Na-Salze eingesetzt.

Neben dem beschriebenen Bleichsystem aus Bleichmitteln, Bleichaktivatoren und gegebenenfalls Bleichkatalysatoren ist für Waschmittel enthaltend die erfindungsgemäss einzusetzenden Co-Tenside auch die Verwendung von Systemen mit enzymatischer Peroxidfreisetzung oder von photoaktivierten Bleichsystemen möglich, siehe z. B. US 4,033,718.

Für eine Reihe von Anwendungsfällen ist es zweckmäßig, wenn die Waschmittel enthaltend die erfindungsgemäss einzusetzenden Co-Tenside Enzyme enthalten. Vorzugsweise in Waschmitteln eingesetzte Enzyme sind Proteasen, Amylasen, Lipasen und Cellulasen. Von den Enzymen werden vorzugsweise mengen von 0,1 bis 1,5 Gew.-%, insbesondere vorzugsweise 0,2 bis 1,0 Gew.-%, des konfektionierten Enzyms zugesetzt. Geeignete Proteasen sind z. B. Savinase und Esperase. Eine geeignete Lipase ist z. B. Lipolase. Eine geeignete Cellulase ist z. B. Celluzym. Auch die Verwendung von Peroxidasen zur Aktivierung des Bleichsystems ist möglich. Man kann einzelne Enzyme oder eine Kombination unterschiedlicher Enzyme einsetzen. Gegebenenfalls kann das Waschmittel enthaltend die erfindungsgemäss einzusetzenden Co-Tenside noch Enzymstabilisatoren, z. B. Calciumpropionat, Natriumformiat oder Borsäuren oder deren Salze, und/oder Oxidationsverhinderer enthalten.

Die Bestandteile von Waschmitteln sind dem Fachmann prinzipiell bekannt. Die obigen und die weiter unten folgenden Listen geeigneter Bestandteile geben nur einen exemplarischen Ausschnitt der bekannten geeigneten Bestandteile wieder.

Die Waschmittel enthaltend die erfindungsgemäss einzusetzenden Co-Tenside können neben den bisher genannten Hauptkomponenten noch folgende weitere übliche Zusätze in den hierfür üblichen Mengen enthalten:

Bekannte Dispergiermittel, wie Naphthalinsulfonsäurekondensate oder Polycarboxilate, Schmutztragemittel, Soil release Agentien, wie Polyetherester, Inkrustationsinhibitoren, pH-regulierende Verbindungen wie Alkalien bzw. Alkalispender (NaOH, KOH, Pentanatriummetasilikat, Natriumcarbonat) oder Säuren (Salzsäure, Phosphorsäure, Amidoschwefelsäure, Citronensäure) Puffersysteme, wie Acetat oder Phosphatpuffer, Ionenaustauscher, Parfüm, Farbstoffe, Vergrauungsinhibitoren, optische (fluoreszierende) Aufheller, Farbübertragungsinhibitoren wie z. B. Polyvinylpyrrolidon, Biozide, wie Isothiazolinone oder 2-Bromo-2-nitro-1,3-propandiol, hydrotrope Verbindungen als Lösungsvermittler bzw. Solubilisatoren, wie Cumolsulfonate, Toluolsulfonate, kurzkettige Fettsäuren, Harnstoff, Alkohole oder Phosphorsäurealkyl/-arylester, Schaumregulatoren zur Stabilisierung oder Dämpfung des Schaums, z. B. Siliconöle, Haut- und Korrosionsschutzmittel, desinfizierende Verbindungen oder Systeme, wie z. B. solche die Chlor oder unterchlorige Säure freisetzen wie Dichlorisocyanurat oder die Iod enthalten, Verdickungsmittel und Stell- und Konfektionierungsmittel.

### Vergrauungsinhibitoren und Soil-Release-Polymere

Geeignete Soil-Release-Polymere und/oder Vergrauungsinhibitoren für Waschmittel sind beispielsweise:
Polyester aus Polyethylenoxiden mit Ethylenglycol und/oder Propylenglycol und aromatischen Dicarbonsäuren oder aromatischen und aliphatischen Dicarbonsäuren;
Polyester aus einseitig endgruppenverschlossenen Polyethylenoxiden mit zwei-und/oder mehrwertigen Alkoholen und Dicarbonsäure.

Derartige Polyester sind bekannt, beispielsweise aus US-A 3,557,039, GB-A 1 154 730, EP-A-185 427, EP-A-241 984, EP-A-241 985, EP-A- 272 033 und US-A 5,142,020.

Weitere geeignete Soil-Release-Polymere sind amphiphile Pfropf- oder Copolymere von Vinyl- und/oder Acrylestern auf Polyalkylenoxide (vgl. US-A 4,746,456, US-A 4,846,995, DE-A-37 11 299, US-A 4,904,408, US-A 4,846,994 und US-A 4,849,126) oder modifizierte Cellulosen wie z. B. Methylcellulose, Hydroxypropylcellulose oder Carboxymethylcellulose.

### Farbübertragungsinhibitoren

Als Farbübertragungsinhibitoren werden beispielsweise Homo- und Copolymere des Vinylpyrrolidons, des Vinylimidazols, des Vinyloxazolidons und des 4-Vinylpyridin-N-oxids mit Molmassen von 15.000 bis 100.000 sowie vernetzte feinteilige Polymere auf Basis dieser Monomeren eingesetzt. Die hier genannte Verwendung solcher Polymere ist bekannt, vgl. DE-B- 22 32 353, DE-A-28 14 287, DE-A-28 14 329 und DE-A-43 16 023.

Geeignete Polyvinylpyridinbetaine sind z. B. in Tai, Formulating Detergents and Personal Care Products, AOCS Press, 2000, Seite 113 beschrieben.

Neben der Anwendung in Wasch- und Reinigungsmitteln für die Textilwäsche im Haushalt sind die erfindungsgemäß verwendbaren Waschmittelzusammensetzungen auch im Bereich der gewerblichen Textilwäsche und der gewerblichen Reinigung einsetzbar. In der Regel wird in diesem Einsatzbereich Peressigsäure als Bleichmittel eingesetzt, die als wäßrige Lösung der Waschflotte zugesetzt wird.

### Verwendung in Textilwaschmitteln

Ein typisches erfindungsgemäss einzusetzendes pulver- oder granulatförmiges Vollwaschmittel kann beispielsweise folgende Zusammensetzung aufweisen:
- 0,5 bis 50 Gew.-%, vorzugsweise 5 bis 30 Gew.-%, mindestens eines anionischen und/- oder nichtionischen Tensids, einschließlich mindestens eines erfindungsgemässen Co-Tensids (Alkylglycidolcarbonat Ic),
- 0,5 bis 60 Gew.-%, vorzugsweise 15 bis 40 Gew.-%, mindestens eines anorganischen Builders,
- 0 bis 20 Gew.-%, vorzugsweise 0,5 bis 8 Gew.-%, mindestens eines organischen Cobuilders,
- 2 bis 35 Gew.-%, vorzugsweise 5 bis 30 Gew.-%, eines anorganischen Bleichmittels,
- 0,1 bis 20 Gew.-%, vorzugsweise 0,5 bis 10 Gew.-%, eines Bleichaktivators, gegebenenfalls in Abmischung mit weiteren Bleichaktivatoren,
- 0 bis 1 Gew.-%, vorzugsweise bis höchstens 0,5 Gew.-%, eines Bleichkatalysators,
- 0 bis 5 Gew.-%, vorzugsweise 0 bis 2,5%, eines polymeren Farbübertragungsinhibitors,
- 0 bis 1,5 Gew.-%, vorzugsweise 0,1 bis 1,0 Gew.-%, Protease,
- 0 bis 1,5 Gew.-%, vorzugsweise 0,1 bis 1,0 Gew.-%, Lipase,
- 0 bis 1,5 Gew.-%, vorzugsweise 0,2 bis 1,0% Gew.-% eines Soil-Release-Polymers,
ad 100% übliche Hilfs- und Begleitstoffe und Wasser.

Vorzugsweise in Waschmitteln eingesetzte anorganische Builder sind Natriumcarbonat, Natriumhydrogencarbonat, Zeolith A und P sowie amorphe und kristalline Na-Silikate sowie Schichtsilikate.

Vorzugsweise in Waschmitteln eingesetzte organische Cobuilder sind Acrylsäure/MaleinsäureCopolymere, Acrylsäure/Maleinsäure/Vinylester- Terpolymere und Citronensäure.

Vorzugsweise in Waschmitteln eingesetzte anorganische Bleichmittel sind Natriumperborat und Natriumcarbonat-Perhydrat.

Vorzugsweise in Waschmitteln eingesetzte anionische Tenside sind lineare und leicht verzweigte Alkylbenzolsulfonate (LAS), Fettalkoholsulfate/ethersulfate und Seifen.

Vorzugsweise in Waschmitteln eingesetzte Enzyme sind Protease, Lipase, Amylase und Cellulase. Von den handelsüblichen Enzymen werden dem Waschmittel in der Regel Mengen von 0,05 bis 2,0 Gew.-%, vorzugsweise 0,2 bis 1,5 Gew.-%, des konfektionierten Enzyms zugesetzt. Geeignete Proteasen sind z.B Savinase, Desazym und Esperase. Eine geeignete Lipasen ist z. B. Lipolase. Eine geeignete Cellulase ist z. B. Celluzym.

Vorzugsweise in Waschmitteln eingesetzte Vergrauungsinhibitoren und Soil-Release-Polymere sind Pfropfpolymere von Vinylacetat auf Polyethylenoxid der Molmasse 2.500-8.000 im Gewichtsverhältnis 1,2:1 bis 3,0:1, Polyethylen-terephthalate/Oxyethylenterephthalate der Molmasse 3.000 bis 25.000 aus Polyethylenoxiden der Molmasse 750 bis 5.000 mit Terephthalsäure und Ethylenoxid und einem Molverhältnis von Polyethylenterephthalat zu Polyoxyethylenterephthalat von 8:1 bis 1:1 sowie Blockpolykondensate gemäß DE-A-44 03 866.

Vorzugsweise in Waschmitteln eingesetzte Farbübertragungsinhibitoren sind lösliche NVP-Homopolymere und/oder Vinylpyrrolidon- und Vinylimidazol-Copolymere mit Molmassen über 5.000.

Die Waschmittel liegen häufig in fester, pulverförmiger Form vor, und enthalten dann in der Regel zusätzlich übliche Stellmittel, die ihnen eine gute Rieselfähigkeit, Dosierbarkeit und Löslichkeit verleihen und die das Zusammenbacken und Stauben verhüten, wie Natriumsulfat oder Magnesiumsulfat.

Pulver- oder granulatförmige Waschmittel enthaltend die erfindungsgemäss einzusetzenden Co-Tenside können bis zu 60 Gew.-% anorganischer Stellmittel enthalten. Vorzugsweise sind diese Waschmittel aber arm an Stellmitteln und enthalten nur bis zu 20 Gew.-%, besonders bevorzugt nur bis 8 Gew.-% an Stellmitteln.

Waschmittel enthaltend die erfindungsgemäss einzusetzenden Co-Tenside können unterschiedliche Schüttdichten im Bereich von 300 bis 1.200, insbesondere 500 bis 950g/l, besitzen. Moderne Kompaktwaschmittel besitzen in der Regel hohe Schüttdichten und zeigen einen Granulataufbau. Kompakt- oder Ultra-Kompaktwaschmittel sowie Extrudate weisen ein Schüttgewicht > 600 g/l auf. Diese gewinnen immer mehr an Bedeutung.

Sofern sie in flüssiger Form eingesetzt werden sollen, können sie als wässrige Mikroemulsionen, Emulsionen oder Lösungen vorliegen. In flüssigen Waschmitteln können zusätzlich Lösungsmittel wie Ethanol, i-Propanol, 1,2-Propylenglykol, oder Butylglykol verwendet werden.

Bei gelförmigen Waschmitteln können zusätzlich Verdicker, wie z. B. Polysaccharide und/oder schwach vernetzte Polycarboxylate (beispielsweise Carbopol® der Fa. Goodrich) eingesetzt werden.

Bei tablettenförmigen Waschmitteln werden zusätzlich Tablettierhilfsmittel wie z. B. Polyethylenglykole mit Molmassen > 1000 g/mol, Polymerdispersionen, und Tablettensprengmittel wie Cellulosederivate, vernetztes Polyvinylpyrrolidon, vernetzte Polyacrylate oder Kombinationen aus Säuren, z. B. Citronensäure + Natriumbicarbonat, um nur einige zu nennen, benötigt.

Ein weiterer Gegenstand der vorliegenden Erfindung ist die Verwendung der Gemische bei der Herstellung von Waschmitteln.

Unter dem Begriff "Haushaltsreiniger" oder "Reiniger" werden im Zusammenhang mit der vorliegenden Erfindung generell Formulierungen verstanden, die zum Reinigen von harten Oberflächen dienen. Sie liegen flüssig, als Gel, Paste oder auch fest vor. Zu den in fester Form gehörenden Materialien gehören Pulver und Kompaktate, wie beispielsweise Granulate und Formkörper, etwa Tabletten. Beispiele umfassen Handgeschirrspülmittel, maschinelle Geschirrreiniger, Metallentfetter, Glasreiniger, Fußbodenreiniger, Allzweckreiniger, Hochdruckreiniger, alkalische Reiniger, saure Reiniger, Spritzentfetter, Molkereireiniger, Polster-, Plastik-, und Badreiniger. Sie enthalten 0,01 bis 40 Gew.-%, bevorzugt 0,1 bis 25 Gew.-% bezogen auf die Gesamtformulierung mindestens einer Substanz der Formeln I und/oder II. Weitere Bestandteile sind nachfolgend aufgeführt.
- ionische Tenside, wie z. B. Alkoholsulfat/-ethersulfaten, Alkylbenzolsulfonate, α-Olefinsulfonate, Sulfosuccinate, wie oben unter "Waschmittel" beschrieben.
- nichtionische Tenside, wie z. B. Alkoholalkoxilate, Alkylaminalkoxilate, Alkylamidethoxilate, Alkylpolyglucoside, wie oben unter "Waschmittel" beschrieben.
- amphotere Tenside, wie z. B. Alkylaminoxide, Betaine, wie oben unter "Waschmittel" beschrieben.
- Builder wie z. B. Polyphosphate, Polycarboxilate, Phosphonate, Komplexbildner, z. B. Methylglycindiessigsäure und deren Salze, Nitrilotriessigsäure und deren Salze, Ethylendiamintetraessigsäure und deren Salze, wie oben unter "Waschmittel" beschrieben.
- Dispergiermittel, wie z. B. Naphthalinsulfonsäurekondensate, Polycarboxilate, wie oben unter "Waschmittel" beschrieben.
- pH-regulierende Verbindungen wie z. B. Alkalien (NaOH, KOH, Pentanatriummetasilikat) oder Säuren (Salzsäure, Phosphorsäure, Amidoschwefelsäure, Citronensäure)
- Enzyme, wie z. B. Lipasen, Amylasen, Proteasen
- Parfüm
- Farbstoffe
- Biozide, wie z. B. Isothiazolinone, 2-Bromo-2-nitro-1,3-propandiol, wie oben unter "Waschmittel" beschrieben.
- Bleichsysteme, bestehend aus Bleichmitteln, wie z. B. Perborat, Percarbonat etc., plus Bleichaktivatoren, wie z. B. Tetraacetylethylendiamin, plus Bleichstabilisatoren, wie oben unter "Waschmittel" beschrieben.
- Solubilisatoren, wie z. B. Cumolsulfonate, Toluolsulfonate, kurzkettige Fettsäuren, Phosphorsäurealkyl/-arylester
- Lösemittel, wie z. B. kurzkettige Alkyloligoglykole wie Butylglykol, Butyldiglykol, Propylenglykolmonomethylether, Alkohole wie Ethanol, i-Propanol, aromatische Lösemittel wie Toluol, Xylol, N-Alkylpyrrolidone, Alkylencarbonate

Die Bestandteile von Reinigern für harte Oberflächen sind dem Fachmann prinzipiell bekannt. Die obige Liste stellt nur einen exemplarischen Ausschnitt der Bestandteile dar. Die Reiniger für harte Oberflächen sind gewöhnlich, aber nicht ausschließlich, wässrig und liegen in der Form von Mikroemulsionen, Emulsionen oder Lösungen vor.

Sollten sie in fester, pulverförmiger Form vorliegen, können zusätzlich Stellmittel wie z. B. Natriumsulfat, Magnesiumsulfat etc. eingesetzt werden.

Bei tablettenförmigen Reinigern werden zusätzlich Tablettierhilfsmittel wie z. B. Polyethylenglykole mit Molmassen > 1000 g/mol, Polymerdispersionen etc., und Tablettensprengmittel wie z. B. Cellulosederivate, vernetztes Polyvinylpyrrolidon, vernetzte Polyacrylate oder Kombinationen aus Säuren, z. B. Citronensäure plus Natriumbicarbonat, um nur einige zu nennen, benötigt.

In einer besonders bevorzugten Ausführungsform der vorliegenden Anmeldung handelt es sich bei den Reinigern um Handgeschirrspülmittel. Ein weiterer Gegenstand der vorliegenden Anmeldung ist daher ein Handgeschirrspülmittel enthaltend mindestens ein Alkylglycidolcarbonat der allgemeinen Formel Ic als Co-Tensid, sowie die Verwendung der Alkylglycidolcarbonate der allgemeinen Formel Ic als Co-Tenside in Handgeschirrspülmitteln.

Produkte aus dem Body-Care-Sektor sind beispielsweise Shampoos, Dusch- und Badegels, Dusch- und Badelotionen, Lippenstifte und kosmetische Formulierungen mit Pflege- und/oder Konditioniereigenschaften wie Stylingprodukte. Beispiele sind Haarschäume, Haargele, Haarsprays oder Nachbehandlungsmittel wie Haarwasser, Lotionen, Kurspülungen, Kurpackungen, Spitzenfluids, Hair-Repair-Mittel, "Hot Oil Treatments", Shampoos, Flüssigseifen, Pflegecremes, Haarfestiger, Haarfärbemittel und Dauerwellmittel. Bei Einsatz in Body-Care-Produkten weisen die Substanzen der Formel I den Vorteil auf, dass die physiologische Reizwirkung der Tensidmischungen abgemildert wird und die Schleimhäute geschützt werden.

Ein weiterer Gegenstand der vorliegenden Erfindung ist die Verwendung von Verbindungen der Formel Ia, worin sowohl A als auch B eine OH-Gruppe sind, als Reinigungstensid.

Ein weiterer Gegenstand der Erfindung ist die Verwendung von Verbindungen der Formel IIIa als Verdicker.

Die Erfindung wird nun in den nachfolgenden Beispielen erläutert.

### Beispiele

### 1. Chlorhydrin der Formel I

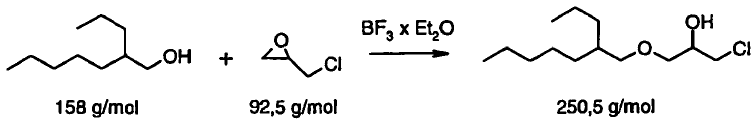

316 g (2 mol) 2-Propylheptanol wurden bei Raumtemperatur zusammen mit 1 g BF₃-Diethyletheratkomplex vorgelegt. Man erwärmte auf 50 °C und dosierte dazu innerhalb von vier Stunden 186 g (2 mol) Epichlorhydrin. Die Mischung wurde noch 30 min bei 50 °C nachgerührt und anschließend auf Raumtemperatur abkühlen lassen. Nach Analyse (GC/MS) war das erwartete Produkt das Hauptprodukt (ca. 60%) der Synthese. Eine Aufreinigung durch Destillation ist möglich.

### 2. Epoxid der Formel Id

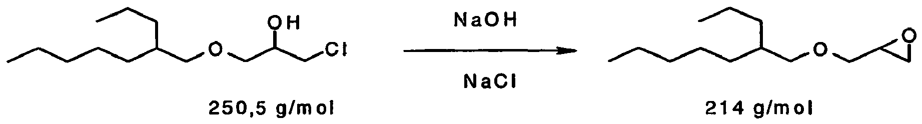

Zu 125.3 g (0.5 mol) des Chlorhydrins tropfte man bei Zimmertemperatur vorsichtig 159 g (1.0 mol) 25% NaOH (in Wasser). Dabei erwärmte man langsam auf 50 °C. Nach beendeter Zugabe erwärmte man weiter auf 100 °C und rührte bei dieser Temperatur für 15 Stunden. Nach dem Abkühlen auf Raumtemperatur trennte man die beiden Phasen. Die obere enthielt das gewünschte Produkt, welches durch Destillation gereinigt werden konnte. Ausbeute: 99%.

### 3. Diol der Formel Ia

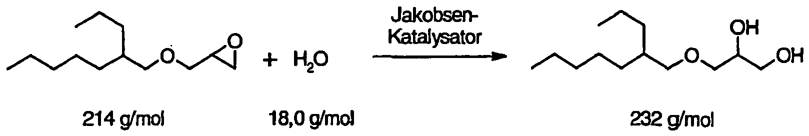

1.55 g (0,0026 mol) N,N'-Bis(3,5-di-tert.-butylsalicyliden)-1,2-cyclohexan-diaminocobalt (II) wurden in 3 ml Toluol vorgelegt. Dazu gab man 30 □1 konz. Essigsäure und rührte bei geöffnetem Kolben 1 h bei Raumtemperatur. Toluol wurde anschließend bei 50 °C und 25 mbar entfernt und der Rückstand 30 min im Vakuum getrocknet. Dazu gab man 35 g (0.128 mol) des Epoxids und tropfte dazu bei <30 °C 3.8 g (0.21 mol) Wasser. Man ließ auf Raumtemperatur abkühlen und rührte bei dieser Temperatur über Nacht. Die braune viskose Flüssigkeit wurde anschließend destilliert und man erhielt 28 g des gewünschten Produkts in einer Reinheit über 90%.

### 4. Alkylglycidolcarbonat der Formel Ic

Die Transformation dieses Produktes in ein Alkylglycidolcarbonat der Formel I kann durchgeführt werden wie in WO 98 00418 beschrieben. 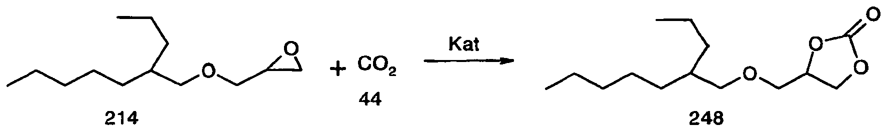 Das Epoxid (40 g, 0.15 mol) wurde zusammen mit einem Katalysator (0.42 g) in Aceton bei Zimmertemperatur vorgelegt. Katalysatoren für die Carbonat-Bildung sind z. B. beschrieben in: Paddock, Nguyen, J. Am. Chem. Soc. 2001, 123, 11498; Kisch, Millini, Wang, Chem. Ber. 1986, 119 (3), 1090; Baba, Nozaki, Matsuda, Bull. Chem. Soc. Jpn. 1987, 60 (4), 1552; Lermontov, Velikokhat'ko, Zavorin, Russ. Chem. Bull. 1998, 47 (7), 1405; Rokicki, Kuran, Pogorzelska-Marciniak, Monatshefte für Chemie 1984, 115, 205. Die Mischung wurde in einem Druckautoklaven auf 110 °C erhitzt und 14 bar CO₂ Druck aufgepresst. Dieser Druck wurde 11 Stunden gehalten, anschließend auf 50 °C abkühlen lassen und entspannt. Alle flüchtigen Anteile wurden am Rotationsverdampfer abgetrennt und das gewünschte Produkt als Sumpf der Destillation erhalten.

### 5. Diol der Formel Ia sowie Umsetzungsprodukte von 2-Propylheptanol mit Glycidol, wobei Glycidol in molarem Überschuss eingesetzt wird

316 g (2 mol) 2-Propylheptanol im Gemisch mit 235 g Xylol (Isomerengemisch) und 2 ml Bortrifluorid-Etherat wurden auf 80 °C unter Rühren erwärmt. Bei dieser Temperatur gab man innerhalb von 4 h 77.0 g (1 mol) Glycidol (96%) zu, rührte noch 1 h bei dieser Temperatur, ließ dann auf Zimmertemperatur abkühlen und filtrierte. Es wurde Wasser zugegeben und dann anschließend das Xylol / Wasser Gemisch am Rotationsverdampfer bei 55 °C im Vakuum evaporiert. Um das Xylol vollständig zu entfernen, wurde gegen Ende der Destillation auf 75 °C erwärmt.

Anschließend entfernte man auch das überschüssige 2-Propylheptanol durch Destillation. Der Rückstand enthielt neben dem Monoaddukt (Diol der Formel I) auch höhere Addukte (Umsetzungsprodukte) des Glycidols an 2-Propylheptanol.

Produkte, die einen höheren durchschnittlichen Glycidol-Anteil haben, sind durch Variation der Stöchiometrie nach analoger Vorschrift zugänglich. Es wurden synthetisiert:
2-Propylheptanol : Glycidol = 1 : 1.5
2-Propylheptanol : Glycidol = 1 : 2
2-Propylheptanol: Glycidol = 1 : 2.7

### 6. Anwendungen

### a) Einsatz des Alkylglycidolcarbonats gemäß Formel I (Beispiel 4) als Co-Tensid Handgeschirrspülmittel

Eine Modellformulierung enthaltend 30 Gew.-% Lutensit® ALBN50 (BASF AG, Alkylbenzolsulfonat, 50%ig), 10 Gew.-% Lutensol® AO7 (BASF AG, C13/15, Alkoholethoxylat, 7Ethylenoxid, 100%ig), 3 Gew.-% 2-Propylheptylglycidolcarbonat (Alkylglycidolcarbonat der Formel Ic) wird mit verschiedenen Mengen Lutensol^{®} A3N (BASF AG, C12,14-Alkoholethoxylat, 3EO, 100%ig BASF AG) versetzt. Die entstehenden Mischungen werden mit einem Uhbelohde-Viskosimeter, Spindel 3, Scherrate 3 s-1 untersucht. In Parallelexperimenten wurde eine entsprechende Tensidmischung, in der das Umsetzungsprodukt gegen Mazox®LDA (Laurylaminoxid, 100%ig, Herkunft BASF Corporation) und gegen Wasser ausgetauscht wurde, untersucht. Die Ergebnisse sind in der Tabelle zusammengefasst. Die Viskositätserhöhung ist bei dem erfindungsgemäßen Produkt am ausgeprägtesten.

| | | | | | | |
|---|---|---|---|---|---|---|
| 0 | 1 | 2 | 4 | 6 | 8 | % Lutensol^{®} A3N |
| 3040 | 3440 | 8200 | 12300 | 18000 | 52000 | 2-Propylheptylglycidolcarbonat |
| | | | | | | (Alkylglycidolcarbonat der Formel Ic) |
| 1210 | 905 | 970 | 1820 | 2890 | 7010 | Wasser |
| 2040 | 2500 | 2910 | 5760 | 12700 | 19200 | Mazox LDA Oxide w.S. |

### b) Einsatz des Alkylglycidolcarbonats gemäß Formel Ic (Beispiel 4) als Co-Tensid Handgeschirrspülmittel

### Schaumstabilisierung mit 2-Propylheptylglycidolcarbonat (Alkylglycidolcarbonat der Formel Ic)

Eine Modellformulierung enthaltend 30 Gew.-% Lutensit® ALBN50 (Alkylbenzolsulfonat, 50%ig), 10 Gew.-% Lutensol® AO7 (C13/15, Alkoholethoxylat, 7 Ethylenoxid, 100%ig), 3 Gew.-% 2-Propylheptylglycidolcarbonat (Alkylglycidolcarbonat der Formel I) und 3 Gew.-% Lutensol A3N (C12,14-Alkoholethoxylat, 3EO, 100%ig) wird auf 2 Gew.-% Tensid verdünnt. In einem Becherglas (51 Volumen, auf 21 gefüllt) wird diese Tensidlösung durch Rühren zum Schäumen versetzt. Wenn sich ein stabiler Zustand eingestellt hat, wird so lange frisches Olivenöl zugetropft, bis der Schaum verschwunden ist. Die dafür notwendige Menge Öl ist ein Maß für die Stabilität des Schaums. In Parallelexperimenten wurde eine entsprechende Tensidmischung, in der das Umsetzungsprodukt gegen Mazox®LDA (Laurylaminoxid, 100%ig) und gegen Wasser ausgetauscht wurde, untersucht. Die Ergebnisse sind in der Tabelle zusammengefasst.

| Zusatz | Verbrauch Olivenöl |
|---|---|
| Propylheptylglycidolcarbonat (Alkylglycidolcarbonat der Formel Ic) | 46 ml |
| Mazox®LDA | 28 ml |
| Wasser | 27 ml |

### c) Einsatz des Diols der Formel Ia als Tensid mit hervorragendem Öllösevermögen (Reinigungstensid)

Auf ein gewogenes Edelstahlblech wird ca. 0,1 g Öl gegeben. Das Blech wird gewogen und in eine Lösung mit 1 g/l des Diols der Formel I gegeben. Es wird die Zeit gemessen, bei der sich der erste Öltopfen abgelöst hat als Maß für die Geschwindigkeit, mit der das Tensid (Diol der Formel I) agiert. Nach 25 Minuten wird das Blech herausgenommen, getrocknet und erneut gewogen. Aus der Differenz zu den früheren Wägungen kann der Anteil des abgelösten Öls bestimmt werden, die als Ölablösevermögen angegeben wird. Die Tabelle zeigt die Wirkung des Diols der Formel I (hergestellt gemäß Beispiel 5) im Vergleich zu einem guten Standardtensid.

| Tensid Ablösezeit in s | Ölablösevermögen in % | |
|---|---|---|
| C_{13,15}-Alkohol + 7EO | 9 | 83 |
| Diol der Formel I | 3 | 83 |

### d) Einsatz der Umsetzungsprodukte IIIa, die einen höheren durchschnittlichen Glycidol-Anteil aufweisen (Hergestellt gemäß Beispiel 5), als Verdickungsmittel

Die Umsetzungsprodukte von 2-Propylheptanol mit Glycidol in unterschiedlichen molaren Verhältnissen (siehe Beispiel 5) wurden in einer Konzentration von 5 gew.% einer Lösung aus C_{13,15}-Alkohols, ethoxyliert (7EO) (Lutensol® AO 7, BASF AG) zugesetzt. Die Viskosität wurde nach der Brookfield-Methode mit Spindel 3 bei 60 1/s bestimmt.

| *Utitsetzungsprodukt* | *Viskosität in cP* |
|---|---|
| 20% C13,15-Alkohol + 7EO, ohne Zusatz | 590 |
| A: 2-Propylheptanol: Glycidol (1:2,7) | 2350 |
| B: 2-Propylheptanol: Glycidol (1:2) | 2590 |
| C: 2-Propylheptanol: Glycidol (1:1,5) | 2090 |
| D: 2-Propylheptanol: Glycidol (1:1) | 731 |

## Patentansprüche

1. Verbindungen der allgemeinen Formel I 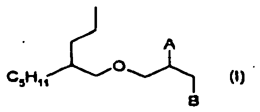 worin
A eine OH-Gruppe und
B eine OH-Gruppe oder ein Halogenatom ist, oder
A und B gemeinsam einen Rest der Formel 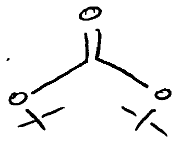 darstellen, oder gemeinsam einen Rest der Formel 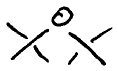 darstellen,
und
C₅H₁₁ ein unverzeigter oder verzweigter C₅H₁₁-Alkylrest oder ein Gemisch aus unverzweigten und verzweigten C₅H₁₁-Alkylresten ist,
wobei 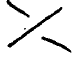 eine Bindung zu einem weiteren Atom bedeutet.

2. Verbindung nach Anspruch 1, **dadurch gekennzeichnet, dass** C₅H₁₁ ein Gemisch aus unverzweigten und verzweigten C₅H₁₁-Alkylresten ist, das 70 bis 99 Gew.-% eines unverzweigten n-C₅H₁₁-Alkylrests und 1 bis 30 Gew.-% eines verzweigten C₅H₁₁-Alkylrests, bevorzugt C₂H₅CH(CH₃)CH₂ und/oder CH₃CH(CH₃)CH₂CH₂, umfasst.

3. Verbindung nach Anspruch 1 oder 2, **dadurch gekennzeichnet, dass** A und B gemeinsam einen Rest der Formel 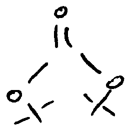 darstellen.

4. Verbindung nach Anspruch 1 oder 2, **dadurch gekennzeichnet, dass** sowohl A als auch B eine OH-Gruppe sind.

5. Umsetzungsprodukt herstellbar durch Umsetzung eines Moläquivalents einer Verbindung gemäß Anspruch 4 mit 1 bis 10 Moläquivalenten 1-Hydroxy-2,3-epoxypropan.

6. Umsetzungsprodukt nach Anspruch 5, **dadurch gekennzeichnet, dass** das Umsetzungsprodukt 1 bis 11, bevorzugt 1 bis 5, besonders bevorzugt 1 bis 2,5 Struktureinheiten der Formel 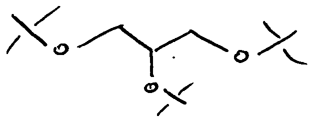 enthält, wobei 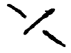 eine Bindung zu einem weiteren Kohlenstoffatom bedeutet.

7. Umsetzungsprodukt herstellbar durch Umsetzung eines Moläquivalents einer Verbindung gemäß Anspruch 1, worin A eine OH-Gruppe und B ein Halogenatom ist, mit 1 bis 10 Moläquivalenten 1-Halogen-2,3-epoxypropan, bevorzugt Epichlorhydrin.

8. Umsetzungsprodukt nach Anspruch 7, **dadurch gekennzeichnet, dass** das Umsetzungsprodukt 1 bis 11, bevorzugt 1 bis 5, besonders bevorzugt 1 bis 2,5 Struktureinheiten der Formel 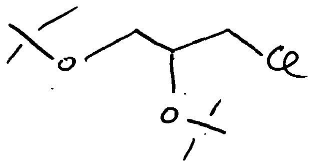 enthält, wobei
eine Bindung zu einem weiteren Atom bedeutet.

9. Verfahren zur Herstellung von Verbindungen der allgemeinen Formel I 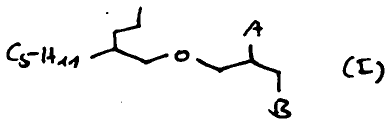 worin
A und B gemeinsam einen Rest der Formel 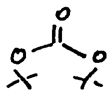 darstellen,
durch Umsetzung einer Verbindung der allgemeinen Formel I,
worin
A und B eine OH-Gruppe sind,
mit Phosgen
oder
durch Umsetzung einer Verbindung der allgemeinen Formel I,
worin
A und B gemeinsam einen Rest der Formel 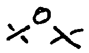 darstellen,
mit CO₂ in Anwesenheit eines Katalysators,
wobei 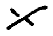 eine Bindung zu einem weiteren Atom bedeutet.

10. Verfahren nach Anspruch 9, **dadurch gekennzeichnet, dass** die Verbindung der allgemeinen Formel I, 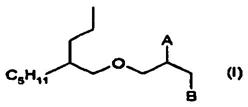 worin A und B gemeinsam einen Rest der Formel
darstellen, durch Umsetzung einer Verbindung der Formel I, worin A eine OH-Gruppe und B ein Halogenatom ist, mit einer Base, wobei eine Bindung zu einem weiteren Kohlenstoffatom bedeutet, hergestellt wird.

11. Verfahren nach Anspruch 10, **dadurch gekennzeichnet, dass** die Verbindung der allgemeinen Formel I 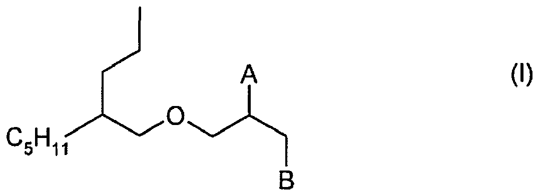 worin A eine OH-Gruppe und B ein Halogenatom ist
und
C₅H₁₁ ein unverzweigter oder verzweigter C₅H₁₁-Alkylrest oder ein Gemisch aus unverzweigten und verzweigten C₅H₁₁-Alkylresten ist,
durch Umsetzung von 2-Propylheptanol mit 1-Halogen-2,3-epoxypropan in Anwesenheit einer Lewis-Säure hergestellt wird.

12. Verfahren nach Anspruch 9, **dadurch gekennzeichnet, dass** die Verbindung der allgemeinen Formel I 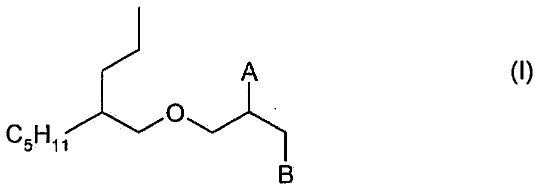 worin A und B eine OH-Gruppe sind, durch Hydrolyse einer Verbindung der allgemeinen Formel I hergestellt wird, worin A und B gemeinsam einen Rest der Formel 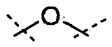 darstellen, in Anwesenheit eines Katalysators, wobei 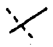 eine Bindung zu einem weiteren Atom bedeutet.

13. Verfahren zur Herstellung eines Umsetzungsproduktes gemäß einem der Ansprüche 5 oder 6, durch Umsetzung eines Moläquivalents einer Verbindung gemäß Anspruch 4 mit 1 bis 10 Moläquivalenten 1-Hydoxy-2,3-epoxypropan.

14. Verfahren zur Herstellung eines Umsetzungsproduktes gemäß einem der Ansprüche 7 oder 8 durch Umsetzung eines Moläquivalents einer Verbindung gemäß Anspruch 1, worin A eine OH-Gruppe und B ein Halogenatom ist, mit 1 bis 10 Moläquivalenten 1-Halogen-2,3-epoxypropan, bevorzugt Epichlorhydrin.

15. Verfahren nach Anspruch 9, umfassend alle oder mehrere der folgenden Schritte:
a) Umsetzung von 2-Propylheptanol mit 1-Halogen-2,3-epoxypropan, wobei eine Verbindung der Formel I 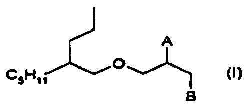 gebildet wird, worin A eine OH-Gruppe und B ein Halogenatom sind,
b) Umsetzung der in Schritt a) gebildeten Verbindung mit einer Base, wobei eine Verbindung der Formel I gebildet wird, worin A und B gemeinsam einen Rest der Formel
darstellen, wobei
eine Bindung zu einem weiteren Atom bedeutet,
und
c) Hydrolyse der in Schritt b) gebildeten Verbindung, wobei eine Verbindung nach Anspruch 4 gebildet wird,
d) Umsetzung der in Schritt d) gebildeten Verbindung mit Phosgen, wobei die gewünschte Verbindung gemäß Anspruch 3 gebildet wird;
oder
e) Umsetzung der in Schritt b) gebildeten Verbindung mit CO₂ in Anwesenheit eines Katalysators, wobei die gewünschte Verbindung gemäß Anspruch 3 gebildet wird.

16. Verfahren nach Anspruch 9, umfassend die folgenden Schritte:
a) Umsetzung von 2-Propylheptanol mit 1-Hydroxy-2,3-epoxypropan (Glycidol), wobei eine Verbindung gemäß Anspruch 4 gebildet wird,
b) Umsetzung der in Schritt a) gebildeten Verbindung mit Phosgen, wobei die gewünschte Verbindung gemäß Anspruch 3 gebildet wird.

17. Verwendung von Verbindungen nach Anspruch 3 oder Mischungen von Verbindungen nach Anspruch 3 als Co-Tenside.

18. Verwendung von Verbindungen nach einem der Ansprüche 5 bis 8 oder Mischungen von Verbindungen nach einem der Ansprüche 5 bis 8 als Verdickungsmittel.

19. Verwendung von Verbindungen nach Anspruch 4 als Reinigungstensid.

20. Haushaltswaschmittel, Haushaltsreiniger, Körperreinigungsmittel oder Körperpflegemittel enthaltend mindestens eine Verbindung nach einem der Ansprüche 3 bis 8.

## Claims

1. A compound of the formula I 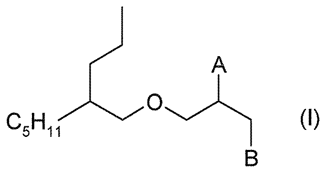 in which
A is an OH group and
B is an OH group or a halogen atom, or
A and B together represent a radical of the formula 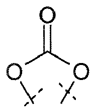 or together represent a radical of the formula 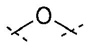 and
C₅H₁₁ is an unbranched or branched C₅H₁₁-alkyl radical or a mixture of unbranched and branched C₅H₁₁-alkyl radicals,
where 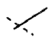 is a bond to a further atom.

2. The compound according to claim 1, wherein C₅H₁₁ is a mixture of unbranched and branched C₅H₁₁-alkyl radicals which comprises 70 to 99% by weight of an unbranched n-C₅H₁₁-alkyl radical and 1 to 30% by weight of a branched C₅H₁₁-alkyl radical, preferably C₂H₅CH(CH₃)CH₂ and/or CH₃CH(CH₃)CH₂CH₂.

3. The compound according to claim 1 or 2, wherein A and B together represent a radical of the formula

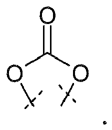

4. The compound according to claim 1 or 2, wherein both A and also B are an OH group.

5. A reaction product which can be produced by reacting one mole equivalent of a compound according to claim 4 with 1 to 10 mol equivalents of 1-hydroxy-2,3-epoxypropane.

6. The reaction product according to claim 5, wherein the reaction product comprises 1 to 11, preferably 1 to 5, particularly preferably 1 to 2.5, structural units of the formula 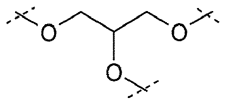 where 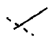 is a bond to a further carbon atom.

7. A reaction product which can be produced by reacting one mole equivalent of a compound according to claim 1, in which A is an OH group and B is a halogen atom, with 1 to 10 mol equivalents of 1-halo-2,3-epoxypropane, preferably epichlorohydrin.

8. The reaction product according to claim 7, wherein the reaction product comprises 1 to 11, preferably 1 to 5, particularly preferably 1 to 2.5, structural units of the formula 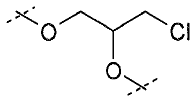 where 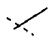 is a bond to a further atom.

9. A process for preparing compounds of the general formula I 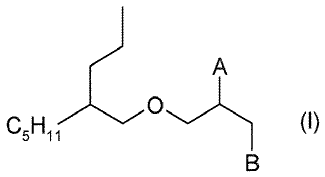 in which A and B together are a radical of the formula 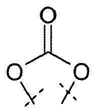 by reacting a compound of the general formula I in which
A and B are an OH group
with phosgene
or
by reacting a compound of the general formula I in which
A and B together are a radical of the formula 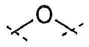 with CO₂ in the presence of a catalyst, where 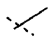 is a bond to a further atom.

10. The method according to claim 9, wherein the compound of the formula I, 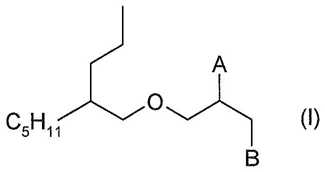 in which A and B together represent a radical of the formula 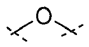 is produced by reacting a compound of the formula I, in which A is an OH group and B is a halogen atom, with a base, where 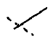 is a bond to a further carbon atom.

11. The method according to claim 10, wherein the compound of the formula I 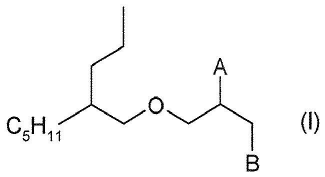 in which A is an OH group and B is a halogen atom, and
C₅H₁₁ is an unbranched or branched C₅H₁₁-alkyl radical or a mixture of unbranched and branched C₅H₁₁-alkyl radicals,
is produced by reacting 2-propylheptanol with 1-halo-2,3-epoxypropane in the presence of a Lewis acid.

12. The method according to claim 9, wherein the compound of the formula I 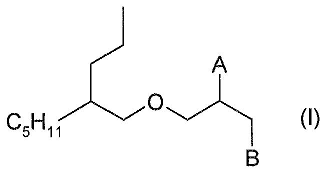 in which
A and B are an OH group
is produced by hydrolysis of a compound of the formula I, in which A and B together represent a radical of the formula 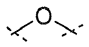 in the presence of a catalyst, where 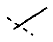 is a bond to a further atom.

13. A method for producing a reaction product according to either of claims 5 and 6 by reacting one mol equivalent of a compound according to claim 4 with 1 to 10 mol equivalents of 1-hydroxy-2,3-epoxypropane.

14. A method for producing a reaction product according to either of claims 7 and 8 by reacting one mol equivalent of a compound according to claim 1, in which A is an OH group and B is a halogen atom, with 1 to 10 mol equivalents of 1-halo-2,3-epoxypropane, preferably epichlorohydrin.

15. The method according to claim 9, comprising all or two or more of the following steps:
a) reaction of 2-propylheptanol with 1-halo-2,3-epoxypropane, where a compound of the formula I 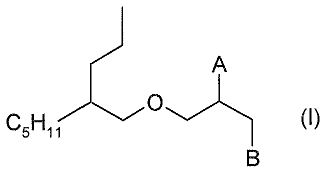 is formed, in which A is an OH group and B is a halogen atom,
b) reaction of the compound formed in step a) with a base, where a compound of the formula I is formed in which A and B together represent a radical of the formula 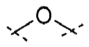 where 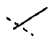 is a bond to a further atom,
and
c) hydrolysis of the compound formed in step b), where a compound according to claim 4 is formed,
d) reaction of the compound formed in step d) with phosgene, where the desired compound according to claim 3 is formed;
or
e) reaction of the compound formed in step b) with CO₂ in the presence of a catalyst, where the desired compound according to claim 3 is formed.

16. The method according to claim 9, comprising the following steps:
a) reaction of 2-propylheptanol with 1-hydroxy-2,3-epoxypropane (glycidol), where a compound according to claim 4 is formed,
b) reaction of the compound formed in step a) with phosgene, where the desired compound according to claim 3 is formed.

17. The use of compounds according to claim 3 or mixtures of compounds according to claim 3 as cosurfactants.

18. The use of compounds according to any of claims 5 to 8 or mixtures of compounds according to any of claims 5 to 8 as thickeners.

19. The use of compounds according to claim 4 as cleaning surfactant.

20. A household detergent, household cleaner, body-cleansing composition or bodycare composition comprising at least one compound according to any of claims 3 to 8.

## Revendications

1. Composés de formule générale I 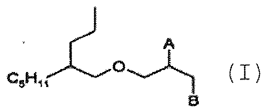 dans laquelle
A est un groupe OH et
B est un groupe OH ou un atome d'halogène, ou
A et B représentent ensemble un radical de formule ou représentent ensemble un radical de formule 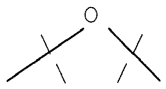 et
C₅H₁₁ est un radical alkyle C₅H₁₁ ramifié ou non ramifié ou un mélange de radicaux alkyle C₅H₁₁ ramifiés et non ramifiés, 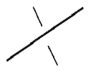
représentant une liaison à un autre atome.

2. Composé selon la revendication 1, **caractérisé en ce que** C₅H₁₁ est un mélange de radicaux alkyle C₅H₁₁ ramifiés et non ramifiés, qui comprend 70 à 99 % en poids d'un radical alkyle n-C₅H₁₁ non ramifié et 1 à 30 % en poids d'un radical alkyle C₅H₁₁ ramifié, de préférence C₂H₅CH(CH₃)CH₂ et/ou CH₃CH(CH₃)CH₂CH₂.

3. Composé selon la revendication 1 ou 2, **caractérisé en ce que** A et B représentent ensemble un radical de formule

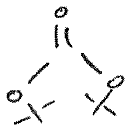

4. Composé selon la revendication 1 ou 2, **caractérisé en ce qu'**aussi bien A que B représentent un groupe OH.

5. Produit de réaction pouvant être préparé par mise en réaction d'un équivalent molaire d'un composé selon la revendication 4 avec 1 à 10 équivalents molaires de 1-hydroxy-2,3-époxypropane.

6. Produit de réaction selon la revendication 5, **caractérisé en ce que** le produit de réaction contient 1 à 11, de préférence 1 à 5, de façon particulièrement préférée 1 à 2,5 motifs structuraux de formule 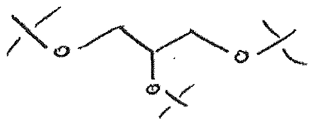 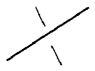 représentant une liaison à un autre atome de carbone.

7. Produit de réaction pouvant être préparé par mise en réaction d'un équivalent molaire d'un composé selon la revendication 1, dans lequel A est un groupe OH et B est un atome d'halogène, avec 1 à 10 équivalents molaires d'un 1-halogéno-2,3-époxypropane, l'épichlorhydrine de préférence.

8. Produit de réaction selon la revendication 7, **caractérisé en ce que** le produit de réaction contient 1 à 11, de préférence 1 à 5, de façon particulièrement préférée 1 à 2,5 motifs structuraux de formule 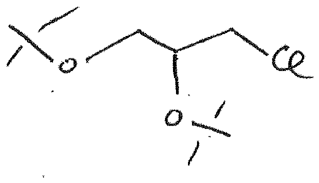 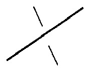 représentant une liaison à un autre atome.

9. Procédé pour la préparation de composés de formule générale I 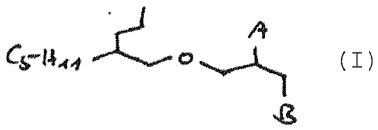 dans laquelle
A et B représentent ensemble un radical de formule 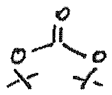 par mise en réaction d'un composé de formule générale I,
dans laquelle
A et B représentent un groupe OH,
avec du phosgène
ou
par mise en réaction d'un composé de formule générale I,
dans laquelle
A et B représentent ensemble un radical de formule 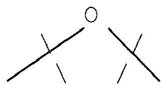 avec CO₂ en présence d'un catalyseur, 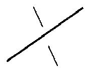 représentant une liaison à un autre atome.

10. Procédé selon la revendication 9, **caractérisé en ce qu'**on prépare le composé de formule générale I 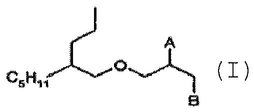 dans laquelle A et B représentent ensemble un radical de formule 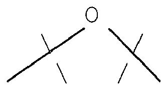 en faisant réagir un composé de formule I, dans laquelle A est un groupe OH et B est un atome d'halogène, avec une base, 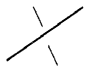 représentant une liaison à un autre atome de carbone.

11. Procédé selon la revendication 10, **caractérisé en ce qu'**on prépare le composé de formule générale I 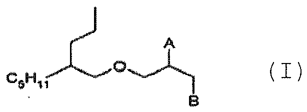 dans laquelle A est un groupe OH et B est un atome d'halogène,
et
C₅H₁₁ est un radical alkyle C₅H₁₁ ramifié ou non ramifié ou un mélange de radicaux alkyle C₅H₁₁ ramifiés et non ramifiés,
en faisant réagir du 2-propylheptanol avec un 1-halogéno-2,3-époxypropane en présence d'un acide de Lewis.

12. Procédé selon la revendication 9, **caractérisé en ce qu'**on prépare le composé de formule générale I 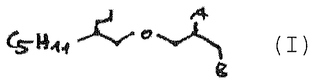 dans laquelle
A et B représentent un groupe OH,
par hydrolyse d'un composé de formule générale I dans lequel A et B représentent ensemble un radical de formule 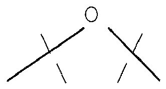 en présence d'un catalyseur, 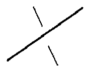 représentant une liaison à un autre atome.

13. Procédé pour la préparation d'un produit de réaction selon l'une quelconque des revendications 5 ou 6, par mise en réaction d'un équivalent molaire d'un composé selon la revendication 4 avec 1 à 10 équivalents molaires de 1-hydroxy-2,3-époxypropane.

14. Procédé pour la préparation d'un produit de réaction selon l'une quelconque des revendications 7 ou 8, par mise en réaction d'un équivalent molaire d'un composé selon la revendication 1, dans lequel A est un groupe OH et B est un atome d'halogène, avec 1 à 10 équivalents molaires d'un 1-halogéno-2,3-époxypropane, l'épichlorhydrine de préférence.

15. Procédé selon la revendication 9, comprenant la totalité ou plusieurs des étapes suivantes :
a) mise en réaction de 2-propylheptanol avec un 1-halogéno-2,3-époxypropane, avec formation d'un composé de formule I 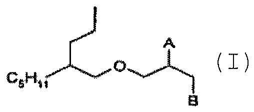 dans laquelle A est un groupe OH et B est un atome d'halogène,
b) mise en réaction du composé formé dans l'étape a) avec une base, avec formation d'un composé de formule I dans lequel A et B représentent ensemble un radical de formule 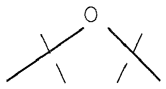 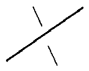 représentant une liaison à un autre atome,
et
c) hydrolyse du composé formé dans l'étape b), avec formation d'un composé selon la revendication 4,
d) mise en réaction du composé formé dans l'étape d) avec du phosgène, de sorte qu'il se forme le composé recherché selon la revendication 3 ;
ou
e) mise en réaction du composé formé dans l'étape b) avec CO₂ en présence d'un catalyseur, de sorte qu'il se forme le composé recherché selon la revendication 3.

16. Procédé selon la revendication 9, comprenant les étapes suivantes :
a) mise en réaction de 2-propylheptanol avec du 1-hydroxy-2,3-époxypropane (glycidol), de sorte qu'il se forme un composé selon la revendication 4,
b) mise en réaction du composé formé dans l'étape a) avec du phosgène, de sorte qu'il se forme le composé recherché selon la revendication 3.

17. Utilisation de composés selon la revendication 3 ou de mélanges de composés selon la revendication 3, en tant que tensioactifs.

18. Utilisation de composés selon l'une quelconque des revendications 5 à 8 ou de mélanges de composés selon l'une quelconque des revendications 5 à 8, en tant qu'épaississants.

19. Utilisation de composés selon la revendication 4, en tant que tensioactif pour nettoyage.

20. Produit de lavage ménager, produit de nettoyage ménager, produit de nettoyage corporel ou produit de soin corporel contenant au moins un composé selon l'une quelconque des revendications 3 à 8.
